# EUROPEAN PATENT APPLICATION

(11) **EP 4 459 376 A2**
(43) Date of publication of application: **06.11.2024**
(21) Application number: 24200469.5
(22) Date of filing: 22.12.2021
(51) Int. Cl.: G03F 7/038

(54) **ALCOHOL COMPOUND, CHEMICALLY AMPLIFIED NEGATIVE RESIST COMPOSITION AND RESIST PATTERN FORMING PROCESS**

(30) Priority: 23.12.2020 JP 2020213114
(62) Divisional of application: 21216712.6
(71) Applicant: Shin-Etsu Chemical Co., Ltd., Tokyo (JP)
(72) Inventor: INOUE, Naoya, Joetsu-shi (JP); WATANABE, Satoshi, Joetsu-shi (JP); KOTAKE, Masaaki, Joetsu-shi (JP)
(74) Representative: Ter Meer Steinmeister & Partner

(57) **Abstract**

A chemically amplified negative resist composition comprising an alcohol compound of specific structure as a crosslinker has a high sensitivity and dissolution contrast and forms a pattern of good profile with reduced values of LER and CDU.

## Description

### CROSS-REFERENCE TO RELATED APPLICATION

This non-provisional application claims priority under 35 U.S.C. §119(a) on Patent Application No. 2020-213114 filed in Japan on December 23, 2020, the entire contents of which are hereby incorporated by reference.

### TECHNICAL FIELD

This invention relates to a novel alcohol compound, a chemically amplified negative resist composition, and a resist pattern forming process.

### BACKGROUND ART

To meet the demand for higher integration density and operating speed of LSIs, the effort to reduce the pattern rule is in rapid progress. The wide-spreading flash memory market and the demand for increased storage capacities drive forward the miniaturization technology. As the advanced miniaturization technology, manufacturing of microelectronic devices at the 65-nm node by the ArF lithography has been implemented in a mass scale. Manufacturing of 45-nm node devices by the next generation ArF immersion lithography is approaching to the verge of high-volume application. One candidate for the next generation 32-nm node devices is ultra-high NA lens immersion lithography using a liquid having a higher refractive index than water in combination with a high refractive index lens and a high refractive index resist film. For forming patterns of smaller feature size, research efforts are now made on resist compositions adapted to shorter wavelength radiation, typically EB and EUV.

The EB lithography is utilized as the ultrafine processing technology and becomes indispensable in processing of photomask blanks to form photomasks for the fabrication of semiconductor devices. Resist compositions for photolithography include positive type compositions adapted to form a pattern by dissolving the exposed region of resist film and negative type compositions adapted to form a pattern while retaining the exposed region of resist film. Either one composition which is easier to use is chosen in accordance with the form of the desired resist pattern.

In general, the method of imagewise writing with EB, in the case of positive type, involves the step of sequentially irradiating EB having a minute area to a region of resist film other than the region to be left, without using a mask. In the case of negative type, the region of resist film to be left is sequentially irradiated. In either case, the operation is to scan the overall region of a processing surface which is divided into minute sections, and thus time-consuming as compared with batch exposure through a photomask. The resist film must have a high sensitivity in order to avoid a lowering of throughput. Also, since the image writing time is long, a difference often develops between the initially written portion and the later written portion and so, the stability with time of the exposed region in vacuum is one of important performance requirements. In the processing of photomask blanks as the important application, some surface materials (for example, a chromium compound, typically chromium oxide film) deposited on photomask substrates are likely to affect the pattern profile of chemically amplified resist film. For obtaining a high resolution and maintaining a profile after etching, it is one of important performance factors to keep the pattern profile of resist film rectangular independent of the type of substrate.

Polymers comprising a major proportion of aromatic structure having an acidic side chain, for example, polyhydroxystyrene are useful in resist materials for the KrF lithography using KrF excimer laser. These polymers are not used in resist materials for the ArF lithography using ArF excimer laser since they exhibit strong absorption at a wavelength of around 200 nm. Yet, these polymers are expected to form useful resist materials for the EB and EUV lithography for forming patterns of smaller feature size than the processing limit of ArF lithography because they offer high etching resistance. See Patent Documents 1 to 3.

In general, chemically amplified negative resist compositions comprise a polymer adapted to dissolve in aqueous alkaline developer and containing hydroxystyrene as the main constituent unit, an acid generator which is decomposed with exposure radiation to generate an acid, and a crosslinker which causes the polymer to crosslink in the presence of the acid serving as a catalyst so that the polymer is insolubilized in the developer (sometimes the crosslinker is integrated with the polymer). Typically, a basic compound is added for controlling the diffusion of the acid generated upon light exposure.

In the photolithography, improvements were made in the control of resist film sensitivity and pattern profile by properly selecting and combining components used in resist compositions and adjusting processing conditions. In the negative resist composition subject to alkaline development, an acid is generated in the exposed region, and the base polymer is insolubilized under the action of the acid. Depending on the structure, a certain polymer is less crosslinkable and thus insufficiently insolubilized, which can lead to degradation of maximum resolution. In addition, the acid generated in the exposed region has high affinity to the alkaline developer so that the exposed region may be dissolved during development. Then the degradation of LER, LWR and CDU becomes a problem. If the acid diffusion is insufficiently controlled, the base polymer is insufficiently insolubilized as a result of diffusion of acid from the exposed region to the unexposed region. Consequently, the lack of resistance to alkaline development invites a top-loss profile. The unexposed region to be originally dissolved away becomes less alkali soluble, leaving scum.

### Citation List

Patent Document 1: JP-A 2006-201532 (US 20060166133, EP 1684118)
Patent Document 2: JP-A 2006-215180
Patent Document 3: JP-A 2008-249762 (USP 9075306, EP 1975711)

### DISCLOSURE OF INVENTION

It is desired to develop an acid-catalyzed chemically amplified negative resist composition capable of forming a pattern, typically line-and-space (LS) pattern with a high sensitivity, reduced values of LER, LWR and CDU, and improved maximum resolution.

An object of the invention is to provide an alcohol compound useful as a crosslinker, a chemically amplified negative resist composition comprising the compound, which when processed by lithography, exhibits a high sensitivity and dissolution contrast and forms a resist pattern of good profile with reduced values of LER, LWR and CDU, and a resist pattern forming process using the resist composition.

The inventors have found that a chemically amplified negative resist composition comprising a crosslinker in the form of an alcohol compound of specific structure and a base polymer, when processed by lithography to form a resist pattern, exhibits a high sensitivity, high dissolution contrast, reduced values of LER, LWR and CDU, controlled dissolution in alkaline developer of a resist film in the exposed region, and satisfactory resolution of isolated lines (IL) and isolated spaces (IS).

In one aspect, the invention provides an alcohol compound having the formula (A1). 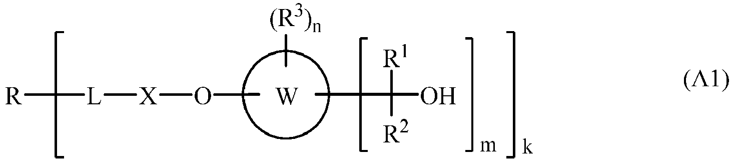 Herein k is an integer of 2 to 4, m is 1 or 2, n is an integer of 0 to 10,
R is oxygen, a C₁-C₁₀ (k+1)-valent aliphatic hydrocarbon group, or an optionally substituted C₆-C₂₀ (k+1)-valent aromatic hydrocarbon group, a constituent -CH₂- in the aliphatic hydrocarbon group may be replaced by -O-, -C(=O)-, -O-C(=O)- or -C(=O)-O-, the atom bonded to L, in the aliphatic or aromatic hydrocarbon group is carbon,
R¹ and R² are each independently hydrogen or a C₁-C₁₀ hydrocarbyl group, some or all of the hydrogen atoms in the hydrocarbyl group may be substituted by halogen, a constituent -CH₂- in the hydrocarbyl group may be replaced by -O- or -C(=O)-, R¹ and R² are not hydrogen at the same time, R¹ and R² may bond together to form an aliphatic ring with the carbon atom to which they are attached,
W is a C₃-C₂₀ group having a mono or polycyclic structure in which a constituent - CH₂- may be replaced by -O-, -S- or -C(=O)-,
R³ is each independently hydrogen or a C₁-C₂₀ hydrocarbyl group which may contain a heteroatom, and when n is at least 2, R³ may be the same or different, or at least two R³ may bond together to form a ring with the atom on W to which they are attached,
L is a single bond, ether bond, ester bond, ketone bond, sulfonic ester bond, carbonate bond or carbamate bond, and
X is a single bond or a C₁-C₄₀ hydrocarbylene group which may contain a heteroatom, with the proviso that X is not a single bond when R is oxygen.

Preferably, the alcohol compound has the formula (A2): 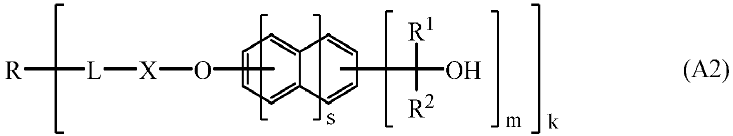 wherein R, R¹, R², L, X, m, and k are as defined above, and s is an integer of 0 to 2.

In another aspect, the invention provides a crosslinker comprising the alcohol compound defined above.

In a further aspect, the invention provides a chemically amplified negative resist composition comprising the crosslinker defined above.

In a preferred embodiment, the resist composition further comprises a base polymer comprising repeat units having the formula (B1) and repeat units having the formula (B2). 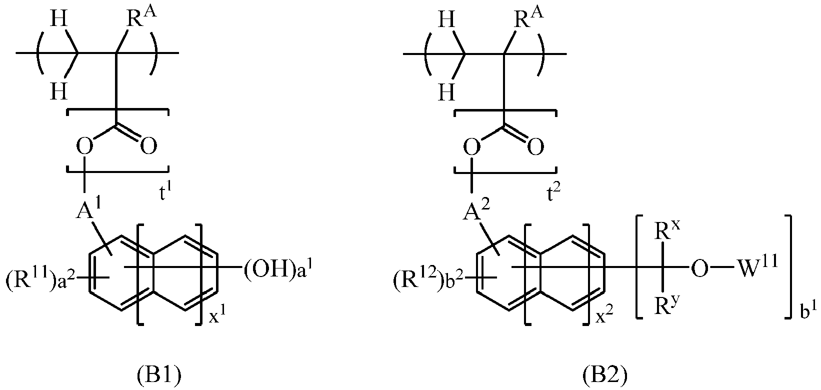 Herein R^{A} is hydrogen, fluorine, methyl or trifluoromethyl,
R¹¹ and R¹² are each independently halogen, an optionally halogenated C₁-C₆ saturated hydrocarbyl group, optionally halogenated C₂-C₈ saturated hydrocarbylcarbonyloxy group, or optionally halogenated C₁-C₆ saturated hydrocarbyloxy group,
A¹ and A² are each independently a single bond or a C₁-C₁₀ saturated hydrocarbylene group in which a constituent -CH₂- may be replaced by -O-,
W¹¹ is hydrogen, a C₁-C₁₀ aliphatic hydrocarbyl group or an optionally substituted aryl group, a constituent -CH₂- in the aliphatic hydrocarbyl group may be replaced by -O-, - C(=O)-, -O-C(=O)- or -C(=O)-O-,
R^{x} and R^{y} are each independently hydrogen or a C₁-C₁₅ saturated hydrocarbyl group which may be substituted with a hydroxy moiety or saturated hydrocarbyloxy moiety, or an optionally substituted aryl group, R^{x} and R^{y} are not hydrogen at the same time, and R^{x} and R^{y} may bond together to form a ring with the carbon atom to which they are attached,
t¹ and t² are each independently 0 or 1, x¹ and x² are each independently an integer of 0 to 2, a² and b¹ are each independently an integer of 1 to 3, a² is an integer satisfying 0 ≤ a² ≤ 5+2x¹-a¹, and b² is an integer satisfying 0 ≤ b² ≤ 5+2x²-b¹.

The base polymer may further comprise repeat units of at least one type selected from repeat units having the formula (B3) and repeat units having the formula (B4). 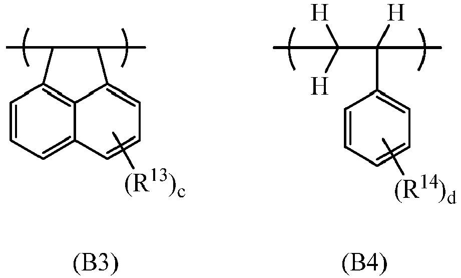 Herein R¹³ is a hydroxy group, halogen atom, acetoxy group, an optionally halogenated C₁-C₈ alkyl group, an optionally halogenated C₁-C₈ saturated hydrocarbyloxy group, an optionally halogenated C₂-C₈ saturated hydrocarbylcarbonyl group, or an optionally halogenated C₂-C₈ saturated hydrocarbylcarbonyloxy group, with the proviso that R¹³ is exclusive of an acid labile group,
R¹⁴ is a halogen atom, acetoxy group, an optionally halogenated C₁-C₈ alkyl group, an optionally halogenated C₁-C₈ saturated hydrocarbyloxy group, an optionally halogenated C₂-C₈ saturated hydrocarbylcarbonyl group, or an optionally halogenated C₂-C₈ saturated hydrocarbylcarbonyloxy group, with the proviso that R¹⁴ is exclusive of an acid labile group,
c and d are each independently an integer of 0 to 4.

The base polymer may further comprise repeat units of at least one type selected from repeat units having the formulae (B5), (B6) and (B7). 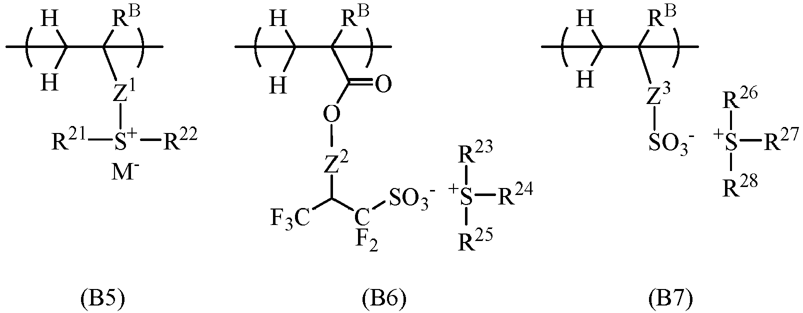 Herein R^{B} is each independently hydrogen or methyl,
Z¹ is a single bond, a C₁-C₆ aliphatic hydrocarbylene group, phenylene group, naphthylene group, or C₇-C₁₈ group obtained by combining the foregoing, or -O-Z¹¹-, - C(=O)-O-Z¹¹- or -C(=O)-NH-Z¹¹-, Z¹¹ is a C₁-C₆ aliphatic hydrocarbylene group, phenylene group, naphthylene group, or C₇-C₁₈ group obtained by combining the foregoing, which may contain a carbonyl moiety, ester bond, ether bond or hydroxy moiety,
Z² is a single bond or -Z²¹-C(=O)-O-, Z²¹ is a C₁-C₂₀ hydrocarbylene group which may contain a heteroatom,
Z³ is a single bond, methylene, ethylene, phenylene, fluorinated phenylene, trifluoromethyl-substituted phenylene group, -O-Z³¹-, -C(=O)-O-Z³¹-, or -C(=O)-NH-Z³¹-, Z³¹ is a C₁-C₁₀ aliphatic hydrocarbylene group, phenylene group, fluorinated phenylene group, trifluoromethyl-substituted phenylene group, or C₇-C₂₀ group obtained by combining the foregoing, which may contain a carbonyl moiety, ester bond, ether bond or hydroxy moiety,
R²¹ to R²⁸ are each independently halogen or a C₁-C₂₀ hydrocarbyl group which may contain a heteroatom, a pair of R²¹ and R²², R²³ and R²⁴ or R²⁶ and R²⁷ may bond together to form a ring with the sulfur atom to which they are attached, and
M⁻ is a non-nucleophilic counter ion.

Typically, the base polymer comprises a polymer containing repeat units having formula (B1) and repeat units having formula (B2), but not repeat units having formula (B5), repeat units having formula (B6), and repeat units having formula (B7).

The resist composition may further comprise an organic solvent, an acid generator, and/or a quencher.

The resist composition may further comprise a fluorinated polymer containing a polymer comprising repeat units having formula (D1) and repeat units of at least one type selected from repeat units having formulae (D2) to (D5).

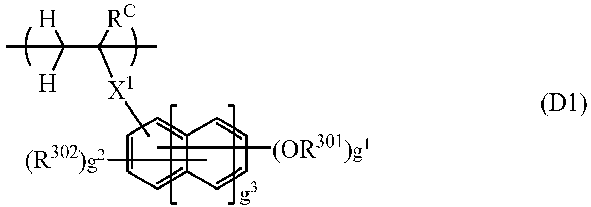

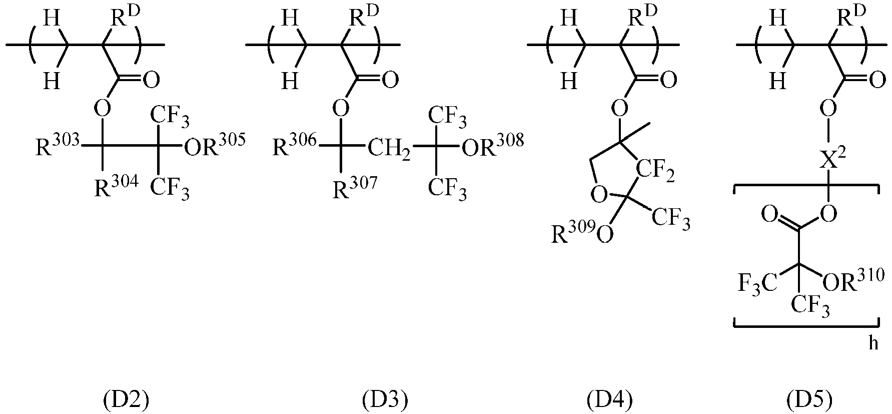

Herein R^{C} is each independently hydrogen or methyl; R^{D} is each independently hydrogen, fluorine, methyl or trifluoromethyl; R³⁰¹ is hydrogen or a C₁-C₅ straight or branched hydrocarbyl group in which a heteroatom-containing moiety may intervene in a carbon-carbon bond; R³⁰² is a C₁-C₅ straight or branched hydrocarbyl group in which a heteroatom-containing moiety may intervene in a carbon-carbon bond; R³⁰³, R³⁰⁴, R³⁰⁶ and R³⁰⁷ are each independently hydrogen or a C₁-C₁₀ saturated hydrocarbyl group; R³⁰⁵, R³⁰⁸, R³⁰⁹ and R³¹⁰ are each independently hydrogen, a C₁-C₁₅ hydrocarbyl group, C₁-C₁₅ fluorinated hydrocarbyl group, or acid labile group, when R³⁰⁵, R³⁰⁸, R³⁰⁹ or R³¹⁰ is a hydrocarbyl or fluorinated hydrocarbyl group, an ether bond or carbonyl moiety may intervene in a carbon-carbon bond; g¹ is an integer of 1 to 3, g² is an integer meeting 0 ≤ g² ≤ 5+2g³-g¹, g³ is 0 or 1, h is an integer of 1 to 3; X¹ is a single bond, -C(=O)-O- or -C(=O)-NH-; and X² is a C₁-C₂₀ (h+1)-valent hydrocarbon group or C₁-C₂₀ (h+1)-valent fluorinated hydrocarbon group.

Also provided is a process for forming a resist pattern comprising the steps of applying the chemically amplified negative resist composition defined herein onto a substrate to form a resist film thereon, exposing patternwise the resist film to high-energy radiation, and developing the exposed resist film in an alkaline developer.

Typically, the high-energy radiation is EB or EUV of wavelength 3 to 15 nm.

### ADVANTAGEOUS EFFECTS OF INVENTION

The alcohol compound is characterized by possessing a secondary or tertiary hydroxy group and functions as a crosslinker. The chemically amplified negative resist composition forms a negative pattern through the mechanisms that when an acid is generated upon exposure, dehydrating reaction of a secondary or tertiary hydroxy group or hydrocarbyloxy group in the alcohol compound takes place to form a cation and an olefin, the cation acting to induce crosslinking reaction to a base polymer and the olefin acting to reduce dissolution in an alkaline developer. In the unexposed region, the alcohol compound exhibits high hydrophilicity by virtue of the hydroxy group. In the exposed region, the alcohol compound undergoes dehydrating reaction under the action of acid generated upon exposure, to promote crosslinking of the base polymer. Thus, the dissolution contrast is high and the exposed region is fully insolubilized. The alcohol compound undergoes dehydrating reaction, that is, polarity switch under the action of acid generated upon exposure, whereby the dissolution in an alkaline developer is reduced. By virtue of these advantages, a chemically amplified negative resist composition having a high sensitivity, improved LER, LWR and CDU, and excellent maximum resolution can be constructed.

### BRIEF DESCRIPTION OF DRAWINGS

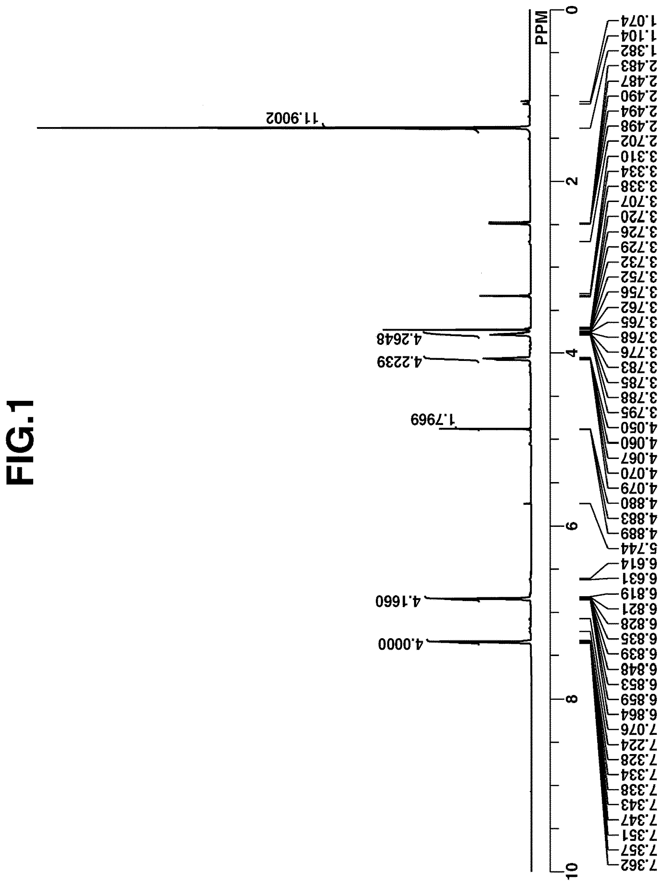

FIG. 1 is a diagram showing ¹H-NMR spectrum of Compound CA-1 in Example 1-1.

### DESCRIPTION OF EMBODIMENTS

As used herein, the singular forms "a," "an" and "the" include plural referents unless the context clearly dictates otherwise. "Optional" or "optionally" means that the subsequently described event or circumstances may or may not occur, and that description includes instances where the event or circumstance occurs and instances where it does not. The notation (Cn-Cm) means a group containing from n to m carbon atoms per group. In chemical formulae, Me stands for methyl, Ac stands for acetyl, and the broken line designates a valence bond. The terms "group" and "moiety" are interchangeable.

The abbreviations and acronyms have the following meaning.
EB: electron beam
EUV: extreme ultraviolet
Mw: weight average molecular weight
Mn: number average molecular weight
Mw/Mn: molecular weight distribution or dispersity
GPC: gel permeation chromatography
PEB: post-exposure bake
PAG: photoacid generator
LER: line edge roughness
LWR: line width roughness
CDU: critical dimension uniformity

### Alcohol compound

The invention provides an alcohol compound having the formula (A1).

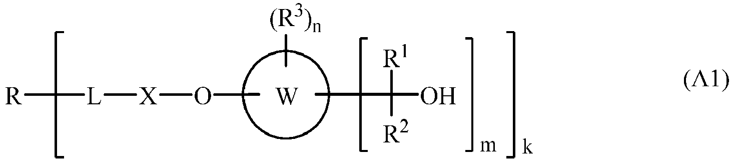

In formula (A1), k is an integer of 2 to 4, m is 1 or 2, and n is an integer of 0 to 10.

R is oxygen, a C₁-C₁₀ (k+1)-valent aliphatic hydrocarbon group, or an optionally substituted C₆-C₂₀ (k+1)-valent aromatic hydrocarbon group, a constituent -CH₂- in the aliphatic hydrocarbon group may be replaced by -O-, -C(=O)-, -O-C(=O)- or -C(=O)-O-. In the aliphatic or aromatic hydrocarbon group, the atom bonded to L is always carbon.

The aliphatic hydrocarbon group R may be saturated or unsaturated and straight, branched or cyclic. Examples thereof include C₁-C₁₀ aliphatic hydrocarbylene groups, and tri- or tetravalent groups obtained by removing one or two hydrogen atoms from the aliphatic hydrocarbylene groups. Suitable aliphatic hydrocarbylene groups include C₁-C₁₀ alkanediyl groups such as methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, 1,1-dimethylethane-1,2-diyl, pentane-1,5-diyl, 2-methylbutane-1,2-diyl, hexane-1,6-diyl, heptane-1,7-diyl, octane-1,8-diyl, nonane-1,9-diyl, and decane-1,10-diyl; C₆-C₁₀ cyclic saturated hydrocarbyl groups such as cyclopentanediyl, methylcyclopentanediyl, ethylcyclopentanediyl, butylcyclopentanediyl, cyclohexanediyl, methylcyclohexanediyl, ethylcyclohexanediyl, butylcyclohexanediyl, norbornanediyl, and adamantanediyl; C₂-C₁₀ alkenediyl groups such as vinylene; and groups obtained by combining the foregoing. Of these, C₁-C₁₀ straight aliphatic hydrocarbon groups are preferred from the standpoints of crosslinking and swell in developer.

Examples of the aromatic hydrocarbon group R include phenyl, tolyl, naphthyl and anthracenyl. Of these, phenyl is preferred for availability of reactants.

In formula (A1), R¹ and R² are each independently hydrogen or a C₁-C₁₀ hydrocarbyl group, some or all of the hydrogen atoms in the hydrocarbyl group may be substituted by halogen, a constituent -CH₂- in the hydrocarbyl group may be replaced by -O- or -C(=O)-. R¹ and R² are not hydrogen at the same time. R¹ and R² may bond together to form an aliphatic ring with the carbon atom to which they are attached.

The hydrocarbyl group represented by R¹ and R² may be saturated or unsaturated and straight, branched or cyclic. Examples thereof include C₁-C₁₀ alkyl groups such as methyl, ethyl, propyl, isopropyl, n-butyl, sec-butyl, tert-butyl, n-pentyl, tert-pentyl, n-hexyl, 2-ethylhexyl, n-octyl, n-nonyl, and n-decyl; C₃-C₁₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, and adamantyl; alkenyl groups such as vinyl; C₆-C₁₀ aryl groups such as phenyl, tolyl and naphthyl; and combinations thereof. Suitable substituted hydrocarbyl groups include oxanorbornyl and fluorophenyl. Preferably, R¹ and R² are the same group, more preferably both methyl.

When R¹ and R² bond together to form an aliphatic ring with the carbon atom to which they are attached, suitable aliphatic rings include cyclopropane, cyclobutane, cyclopentane and cyclohexane rings, but are not limited thereto.

In formula (A1), R³ is each independently hydrogen or a C₁-C₂₀ hydrocarbyl group which may contain a heteroatom. The hydrocarbyl group may be saturated or unsaturated and straight, branched or cyclic. Examples thereof include C₁-C₂₀ alkyl groups such as methyl, ethyl, propyl, isopropyl, n-butyl, sec-butyl, tert-butyl, n-pentyl, tert-pentyl, n-hexyl, n-octyl, 2-ethylhexyl, n-nonyl, and n-decyl; C₃-C₂₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decanyl, adamantyl, and adamantylmethyl; C₆-C₂₀ aryl groups such as phenyl, naphthyl and anthracenyl; and combinations thereof. In the hydrocarbyl group, some or all of the hydrogen atoms may be substituted by a moiety containing a heteroatom such as oxygen, sulfur, nitrogen or halogen, and a constituent -CH₂- may be replaced by a moiety containing a heteroatom such as oxygen, sulfur or nitrogen, so that the group may contain a hydroxy, fluorine, chlorine, bromine, iodine, cyano, carbonyl, ether bond, ester bond, sulfonic ester bond, carbonate bond, carbamate bond, amide bond, imide bond, lactone ring, sultone ring, thiolactone ring, lactam ring, sultam ring, carboxylic anhydride or haloalkyl moiety.

When n is 2 or more, a plurality of R³ may be the same or different, or at least two R³ may bond together to form a ring with the atom on W to which they are attached. Examples of the ring thus formed include cyclopropane, cyclobutane, cyclopentane, cyclohexane, norbornane, and adamantane rings. It is acceptable that two R³ bond to a common carbon atom and bond to each other to form a spiro ring structure with W. Among others, R³ is preferably hydrogen for availability of reactants.

In formula (A1), W is a C₃-C₂₀ group having a mono or polycyclic structure in which a constituent -CH₂- may be replaced by -O-, -S- or -C(=O)-. Examples of the group W are shown below, but not limited thereto.

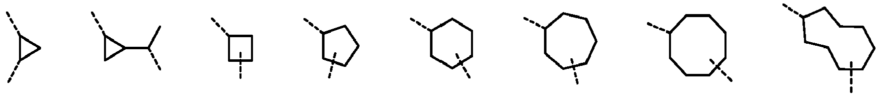

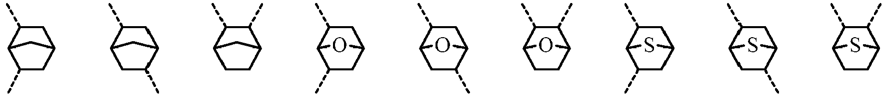

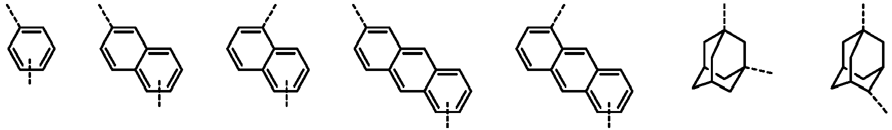

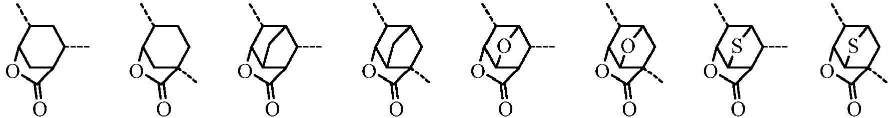

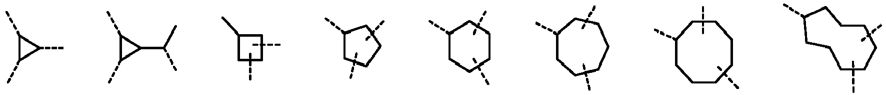

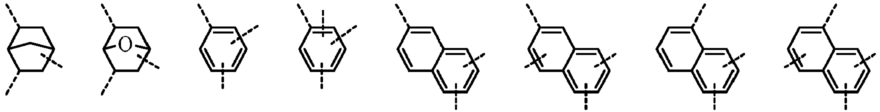

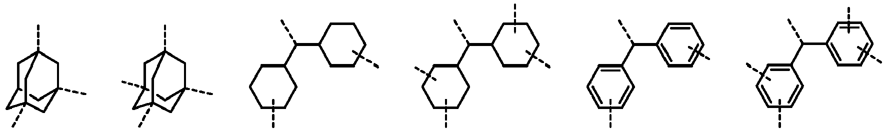

Among others, W is preferably a group having a cyclohexane, benzene or naphthalene ring for availability of reactants.

In formula (A1), L is a single bond, ether bond, ester bond, ketone bond, sulfonic ester bond, carbonate bond or carbamate bond. Preferably L is a single bond or ester bond.

In formula (A1), X is a single bond or a C₁-C₄₀ hydrocarbylene group which may contain a heteroatom. Examples of the C₁-C₄₀ hydrocarbyl group which may contain a heteroatom are shown below, but not limited thereto. It is noted that the asterisk (*) designates a point of attachment to L or O.

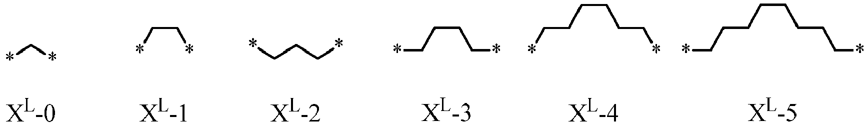

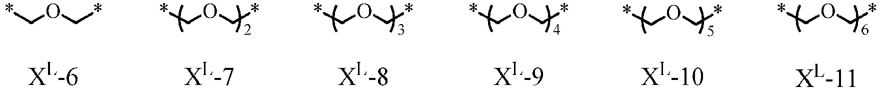

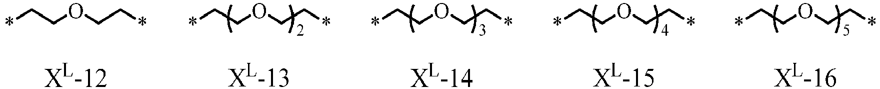

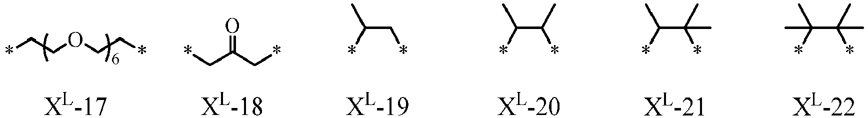

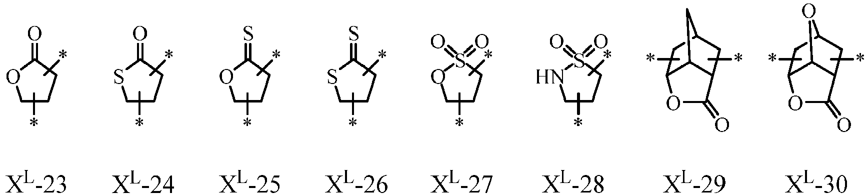

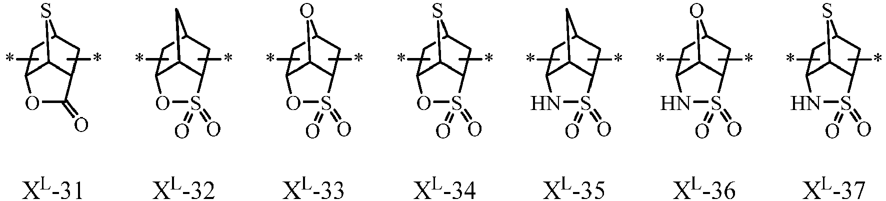

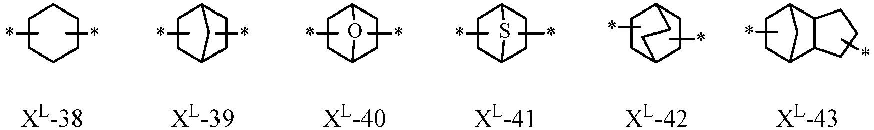

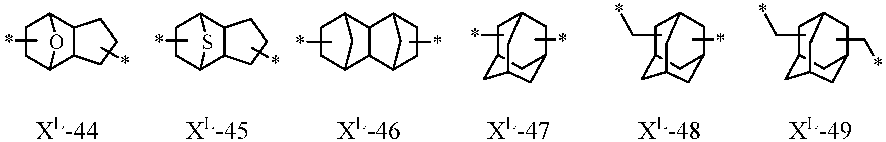

Of these, preference is given to X^{L}-0 to X^{L}-22 and X^{L}-47 to X^{L}-49, with X^{L}-0 to X^{L}-17 being more preferred.

Of the alcohol compounds having formula (A1), those having the formula (A2) are preferred. 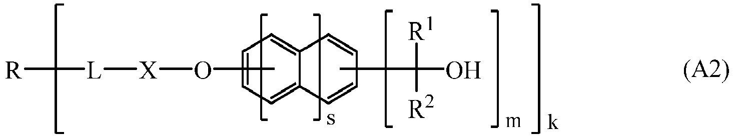 Herein R, R¹, R², L, X, m, and k are as defined above, and s is an integer of 0 to 2.

Illustrative examples of the alcohol compound having formula (A1) are shown below, but not limited thereto. Herein, p is an integer of 1 to 8.

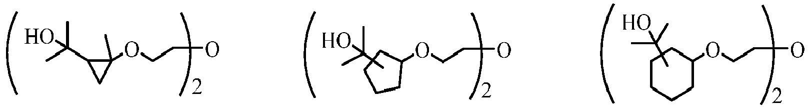

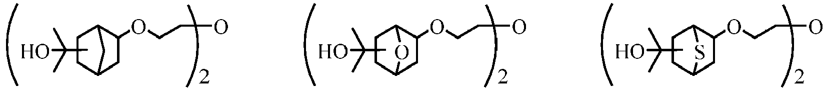

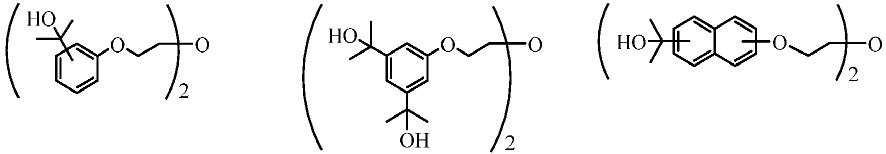

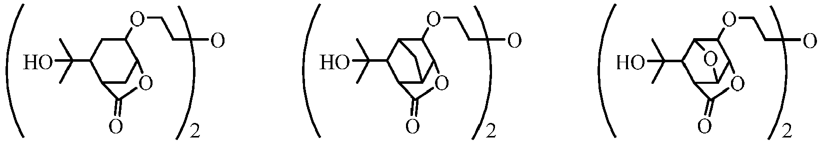

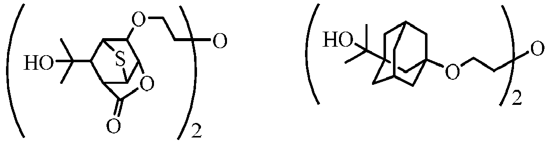

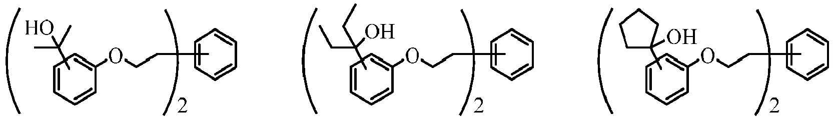

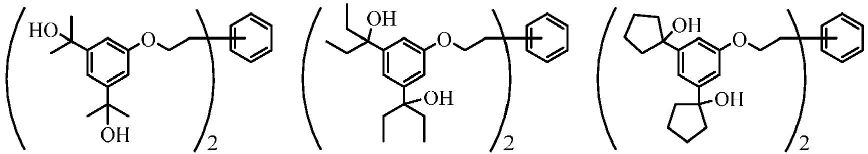

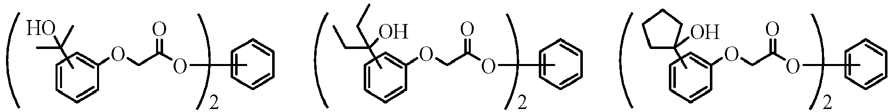

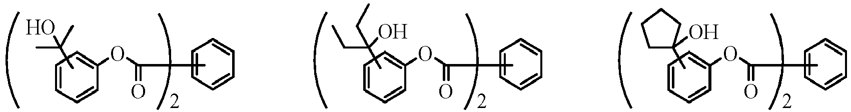

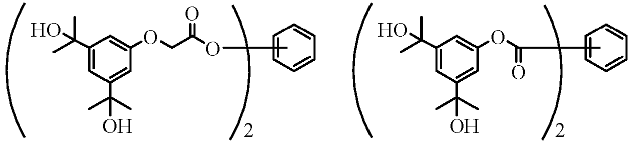

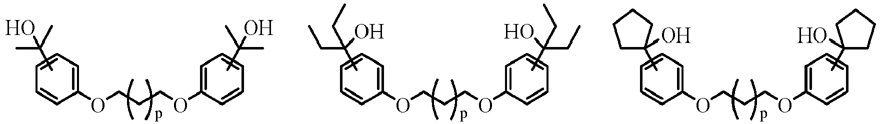

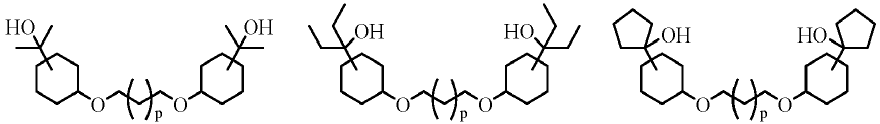

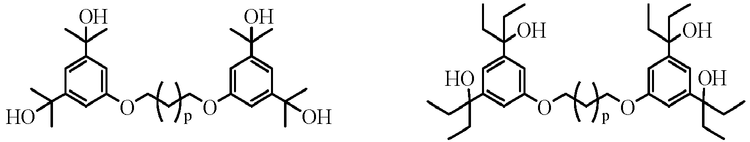

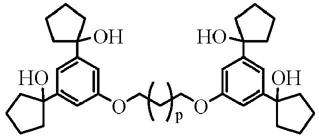

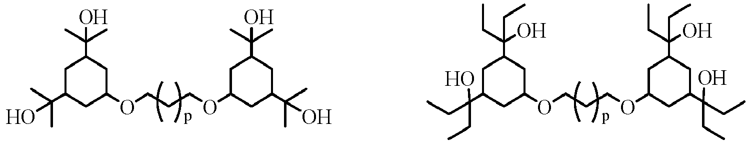

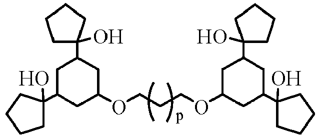

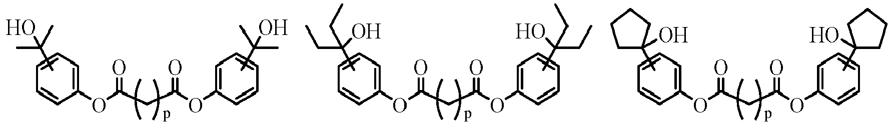

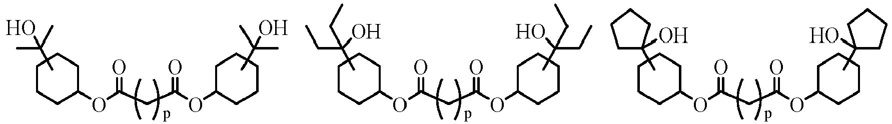

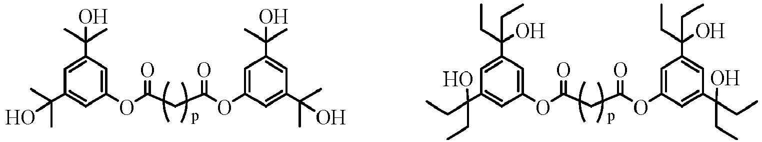

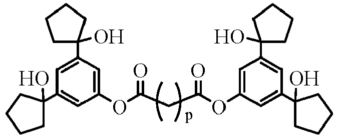

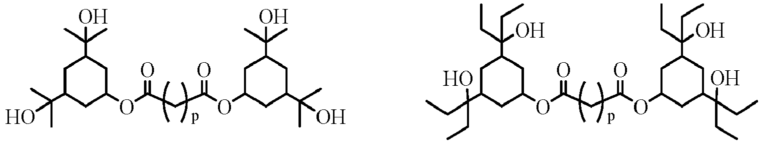

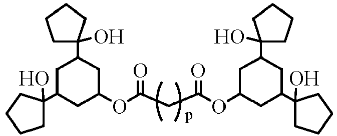

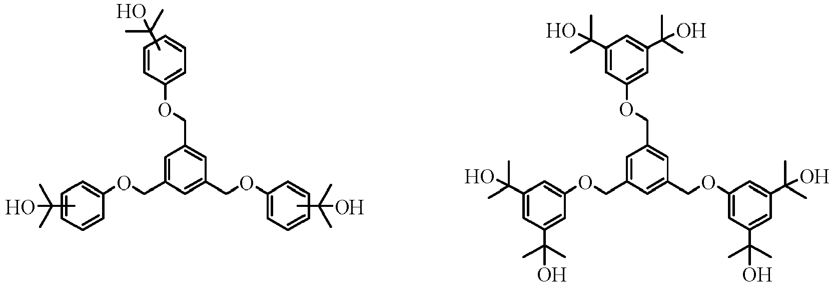

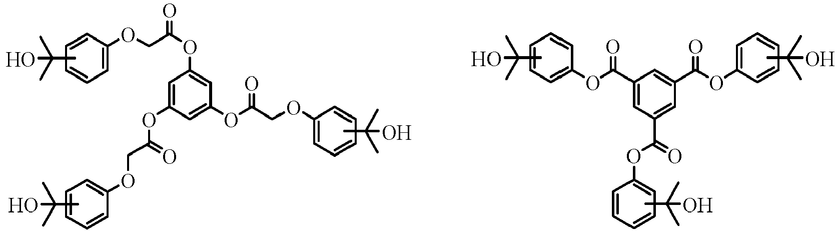

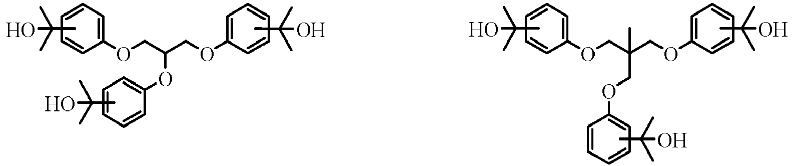

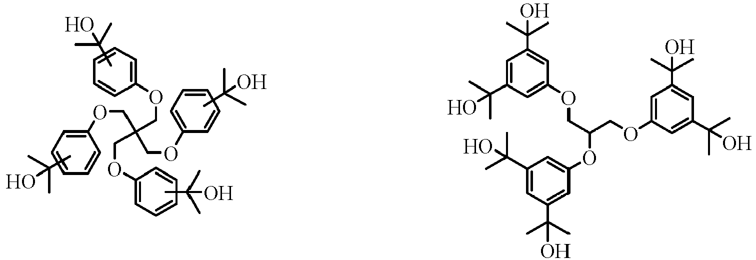

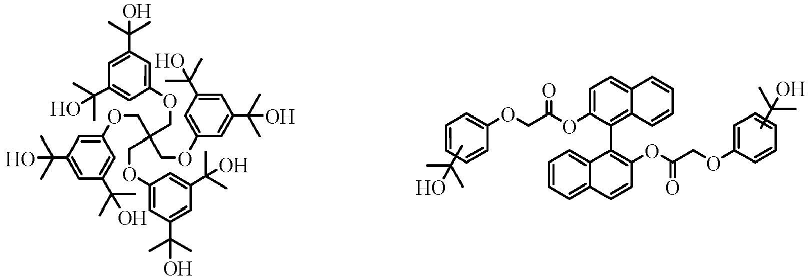

The alcohol compound having formula (A1) can be synthesized, for example, according to the following scheme A, B or C. Although reference is made to the synthesis of a compound having the formula (A1-1), also referred to as compound A1-1, a compound having the formula (A1-2), also referred to as compound A1-2, and a compound having the formula (A1-3), also referred to as compound A1-3, as typical examples, the synthesis method is not limited thereto.

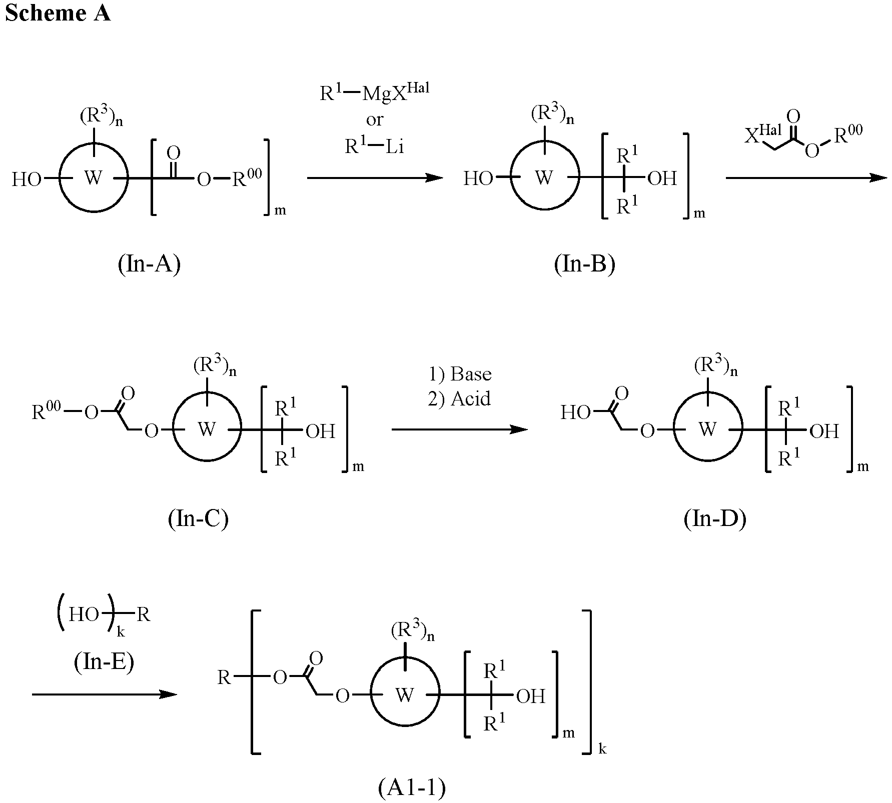

Herein R, W, R¹, R³, k, m, and n are as defined above, R⁰⁰ is a C₁-C₃ hydrocarbyl group, and X^{Hal} is a halogen atom other than fluorine.

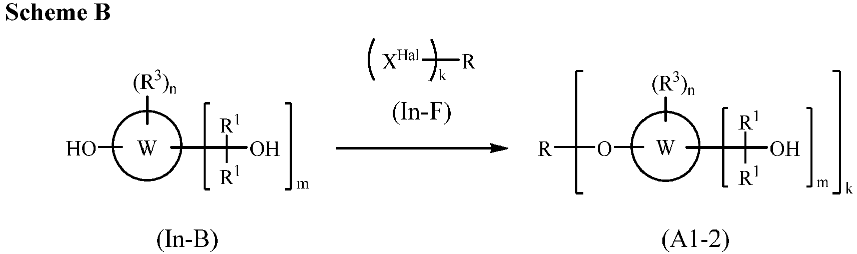

Herein R, W, R¹, R³, k, m, n, and X^{Hal} are as defined above.

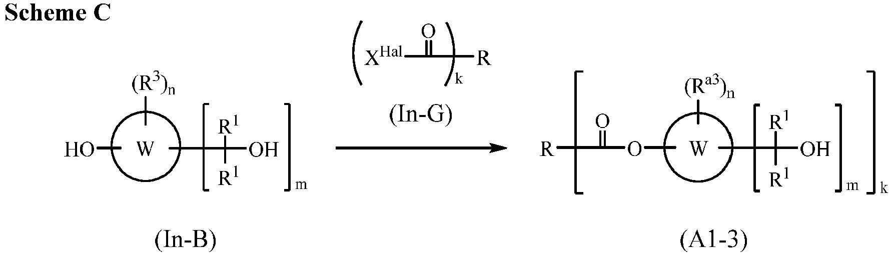

Herein R, W, R¹, R³, k, m, n, and X^{Hal} are as defined above.

Scheme A illustrates the process for the synthesis of compound A1-1, including the first step of reacting an intermediate In-A, which is commercially available or can be synthesized by any well-known organic synthesis method, with a Grignard reagent or organic lithium reagent to form an intermediate In-B. The reaction may be performed by any well-known organic synthesis method. Specifically, the reaction is performed by diluting the Grignard reagent or organic lithium reagent with a solvent such as tetrahydrofuran (THF) or diethyl ether, and adding dropwise a solution of intermediate In-A in a solvent such as THF or diethyl ether thereto. The amount of the Grignard reagent or organic lithium reagent used is preferably (2m¹+2) equivalents in consideration of the number of ester bonds in intermediate In-A and the amount of deactivation by hydroxy group. The reaction temperature preferably ranges from room temperature to approximately the boiling point of the solvent. It is desirable from the standpoint of yield that the reaction time is determined so as to drive the reaction to completion by monitoring the reaction process by gas chromatography (GC) or silica gel thin layer chromatography (TLC). Usually, the reaction time is about 30 minutes to 2 hours. From the reaction mixture, the intermediate In-B is recovered through an ordinary aqueous workup. If necessary, the intermediate In-B may be purified by a standard technique such as chromatography or recrystallization.

The second step is to react intermediate In-B with a haloacetate compound to form an intermediate In-C. The reaction may be performed by any well-known organic synthesis method. Specifically, the reaction is performed by dissolving intermediate In-B and a base in a polar aprotic solvent such as N,N-dimethylformamide (DMF) or dimethyl sulfoxide (DMSO), and adding dropwise the haloacetate compound thereto. Examples of the base used herein include inorganic bases such as potassium carbonate and cesium carbonate and organic bases such as triethylamine and N,N-diisopropylethylamine. In the case of the haloacetate compound whose halogen is chlorine or bromine, the reaction rate may be accelerated by adding a catalytic amount of sodium iodide or potassium iodide. The reaction temperature preferably ranges from room temperature to 100°C. It is desirable from the standpoint of yield that the reaction time is determined so as to drive the reaction to completion by monitoring the reaction process by GC or TLC. Usually, the reaction time is about 2 to 12 hours. From the reaction mixture, the intermediate In-C is recovered through an ordinary aqueous workup. If necessary, the intermediate In-C may be purified by a standard technique such as chromatography or recrystallization.

The third step is hydrolysis of the intermediate In-C to form an intermediate In-D. The reaction may be performed by any well-known organic synthesis method. Specifically, the reaction is performed by dissolving intermediate In-C in 1,4-dioxane or the like and adding dropwise a base thereto. Examples of the base used herein include inorganic bases such as sodium hydroxide and potassium hydroxide. The reaction temperature preferably ranges from room temperature to 600°C. It is desirable from the standpoint of yield that the reaction time is determined so as to drive the reaction to completion by monitoring the reaction process by TLC. Usually, the reaction time is about 2 to 12 hours. At the end of reaction, an acid is added to the reaction mixture to quench the reaction. Suitable acids include aqueous solutions such as hydrochloric acid and nitric acid. From the reaction mixture, the intermediate In-D is recovered through an ordinary aqueous workup. If necessary, the intermediate In-D may be purified by a standard technique such as chromatography or recrystallization.

The fourth step is reaction of the intermediate In-D with an intermediate In-E to form a compound A1-1. When an ester bond is directly formed from the carboxy group of intermediate In-D and the hydroxy group of intermediate In-E, a variety of condensing agents may be used. Suitable condensing agents include N,N'-dicyclohexylcarbodiimide, N,N'-diisopropylcarbodiimide, 1-[3-(dimethylamino)propyl]-3-ethylcarbodiimide, and 1-ethyl-3-(3-dimethylaminopropyl)carbodiimide hydrochloride. From the standpoint of ease of removal of a urea compound formed as a by-product at the end of reaction, it is preferred to use 1-ethyl-3-(3-dimethylaminopropyl)carbodiimide hydrochloride. Specifically, the reaction is performed by dissolving intermediate In-D and intermediate In-E in a halogenated solvent such as methylene chloride and adding a condensing agent thereto. The reaction rate may be accelerated by adding N,N-dimethyl-4-dimethylaminopyridine as a catalyst. It is desirable from the standpoint of yield that the reaction time is determined so as to drive the reaction to completion by monitoring the reaction process by TLC. Usually, the reaction time is about 12 to 24 hours. At the end of reaction, the urea compound as by-product is removed by filtration or water washing if necessary. From the reaction mixture, the target compound A1-1 is recovered through an ordinary aqueous workup. If necessary, the compound A1-1 may be purified by a standard technique such as chromatography or recrystallization.

Scheme B illustrates the process for the synthesis of compound A1-2, including the step of reacting an intermediate In-B with a haloalkyl intermediate In-F. The reaction may be performed by any well-known organic synthesis method. Specifically, the reaction is performed by dissolving intermediate In-B and a base in a polar aprotic solvent such as DMF or DMSO, and adding dropwise a solution of the haloalkyl intermediate In-F in the same solvent thereto. Examples of the base used herein include inorganic bases such as potassium carbonate and cesium carbonate and organic bases such as triethylamine and N,N-diisopropylethylamine. In the case of intermediate In-F whose terminal halogen is chlorine or bromine, the reaction rate may be accelerated by adding a catalytic amount of sodium iodide or potassium iodide. The reaction temperature preferably ranges from room temperature to 100°C. It is desirable from the standpoint of yield that the reaction time is determined so as to drive the reaction to completion by monitoring the reaction process by GC or TLC. Usually, the reaction time is about 2 to 12 hours. From the reaction mixture, the compound A1-2 is recovered through an ordinary aqueous workup. If necessary, the compound A1-2 may be purified by a standard technique such as chromatography or recrystallization.

Scheme C illustrates the process for the synthesis of compound A1-3, including the step of reacting an intermediate In-B with a haloacyl intermediate In-G. The reaction may be performed by any well-known organic synthesis method. Specifically, the reaction is performed by dissolving intermediate In-B and a base in a polar aprotic solvent such as DMF or DMSO, and adding dropwise a solution of the haloacyl intermediate In-G in the same solvent thereto. Examples of the base used herein include inorganic bases such as potassium carbonate and cesium carbonate and organic bases such as triethylamine and N,N-diisopropylethylamine. The reaction temperature preferably ranges from room temperature to 100°C. It is desirable from the standpoint of yield that the reaction time is determined so as to drive the reaction to completion by monitoring the reaction process by GC or TLC. Usually, the reaction time is about 2 to 12 hours. From the reaction mixture, the compound A1-3 is recovered through an ordinary aqueous workup. If necessary, the compound A1-3 may be purified by a standard technique such as chromatography or recrystallization.

The preparation methods according to Schemes A to C are merely exemplary, and the method for preparing the inventive alcohol compound is not limited thereto. Although Schemes A to C refer to the synthesis of compounds having an ester bond or ether bond, it will occur to the skilled artisan to synthesize compounds having a sulfonic ester bond, carbonate bond or carbamate bond using any known organic chemistry method.

### Chemically amplified negative resist composition

The invention also provides a chemically amplified negative resist composition comprising essentially a crosslinker in the form of the alcohol compound defined above. Preferably, the resist composition contains a base polymer of specific structure in addition to the alcohol compound.

In the chemically amplified negative resist composition, the crosslinker in the form of the alcohol compound is preferably present in an amount of 0.01 to 40 parts by weight, more preferably 0.05 to 10 parts by weight per 80 parts by weight of the base polymer, in view of sensitivity and acid diffusion controlling effect.

### Base polymer

The base polymer in the resist composition is defined as comprising repeat units having the following formula (B1), also referred to as repeat units (B1), and repeat units having the following formula (B2), also referred to as repeat units (B2).

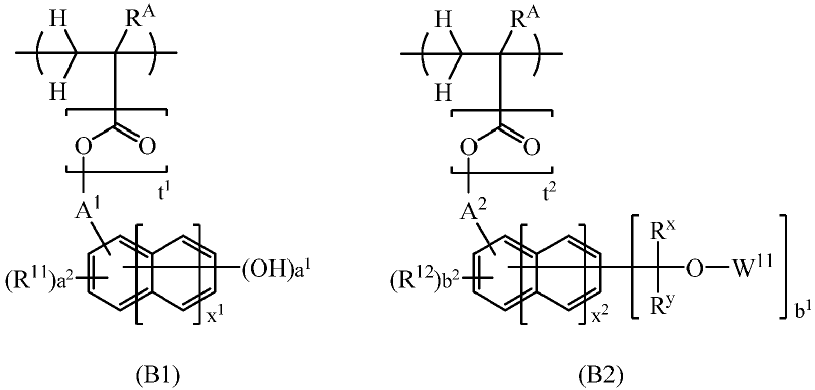

The repeat units (B1) are units for establishing etch resistance and for providing adhesion to substrates and dissolution in alkaline developer. The repeat units (B2) are units for allowing the acid labile group to undergo elimination reaction under the action of acid which is generated from the acid generator upon exposure to high-energy radiation, for thereby inducing alkaline insolubilization and crosslinking reaction among polymer molecules. The repeat units (B2) act to promote the progress of negative reaction to enhance resolution performance.

In formulae (B1) and (B2), R^{A} is hydrogen, fluorine, methyl or trifluoromethyl.

R¹¹ and R¹² are each independently halogen, an optionally halogenated C₁-C₆ saturated hydrocarbyl group, optionally halogenated C₂-C₈ saturated hydrocarbylcarbonyloxy group, or optionally halogenated C₁-C₆ saturated hydrocarbyloxy group. The saturated hydrocarbyl group and the saturated hydrocarbyl moiety in the saturated hydrocarbylcarbonyloxy group and saturated hydrocarbyloxy group may be straight, branched or cyclic. Examples thereof include alkyl groups such as methyl, ethyl, propyl, isopropyl, butyl, pentyl and hexyl, cycloalkyl groups such as cyclopropyl, cyclobutyl, cyclopentyl and cyclohexyl, and a combination thereof. As long as the carbon count does not exceed the upper limit, the polymer is fully dissolvable in alkaline developer.

In formulae (B 1) and (B2), A¹ and A² are each independently a single bond or a C₁-C₁₀ saturated hydrocarbylene group in which a constituent -CH₂- may be replaced by -O-. The saturated hydrocarbylene group may be straight, branched or cyclic and examples thereof include alkanediyl groups such as methylene, ethane-1,1-diyl, ethane-1,2-diyl, propane-1,2-diyl, propane-1,3-diyl, butane-1,4-diyl, pentane-1,5-diyl, and hexane-1,6-diyl; cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of t¹=1 in formula (B 1), the ether bond may be situated at any position excluding the position between the carbon at α-position and the carbon at β-position relative to the ester oxygen. In case of t¹=0, the atom attached to the main chain becomes the ether oxygen atom, and a second ether bond may be situated at any position excluding the position between the carbon at α-position and the carbon at β-position relative to the ether oxygen atom. Since the carbon count of the saturated hydrocarbylene group is 10 or less, the polymer is fully dissolvable in alkaline developer.

In formula (B2), W¹¹ is hydrogen, a C₁-C₁₀ aliphatic hydrocarbyl group or an optionally substituted aryl group. The aliphatic hydrocarbyl group may be straight, branched or cyclic and examples thereof include alkyl groups such as methyl, ethyl, propyl, isopropyl, and cyclic aliphatic hydrocarbyl groups such as cyclopentyl, cyclohexyl and adamantyl. Typical of the aryl group is phenyl. A constituent -CH₂- in the aliphatic hydrocarbyl group may be replaced by -O-, -C(=O)-, -O-C(=O)- or -C(=O)-O-. The constituent -CH₂- in the hydrocarbyl group may bond to the oxygen atom in formula (B2). Typical of the group after such replacement is methylcarbonyl.

In formula (B2), R^{x} and R^{y} are each independently hydrogen, or a C₁-C₁₅ saturated hydrocarbyl group which may be substituted with a hydroxy moiety or saturated hydrocarbyloxy moiety, or an optionally substituted aryl group. Both R^{x} and R^{y} are not hydrogen at the same time. R^{x} and R^{y} may bond together to form a ring with the carbon atom to which they are attached. The saturated hydrocarbyl group may be straight, branched or cyclic and examples thereof include alkyl groups such as methyl, ethyl, propyl, butyl and structural isomers thereof, and substituted forms of these groups in which some hydrogen is substituted by a hydroxy moiety or saturated hydrocarbyloxy moiety.

In formulae (B1) and (B2), t¹ and t² are each independently 0 or 1. The subscripts x¹ and x² are each independently an integer of 0 to 2, and the relevant structure represents a benzene skeleton in case of x¹ or x²=0, a naphthalene skeleton in case of x¹ or x²=1, and an anthracene skeleton in case of x¹ or x²=2. The subscripts a¹ and b¹ are each independently an integer of 1 to 3, a² is an integer satisfying 0 ≤ a² ≤ 5+2x¹-a¹, and b² is an integer satisfying 0 ≤ b² ≤ 5+2x²-b¹. In case of x¹=0, preferably a¹ is an integer of 1 to 3 and a² is an integer of 0 to 3; in case of x¹=1 or 2, preferably a¹ is an integer of 1 to 3 and a² is an integer of 0 to 4. In case of x²=0, preferably b¹ is an integer of 1 to 3 and b² is an integer of 0 to 3; in case of x²=1 or 2, preferably b¹ is an integer of 1 to 3, and b² is an integer of 0 to 4.

In the embodiment wherein t¹=0 and A¹ is a single bond, that is, the aromatic ring directly bonds to the polymer main chain, or differently stated, the unit is free of the linker (-CO-O-A¹-), the repeat units (B1) are preferably repeat units having the following formula (B1-1), which are also referred to as repeat units (B 1-1), hereinafter. 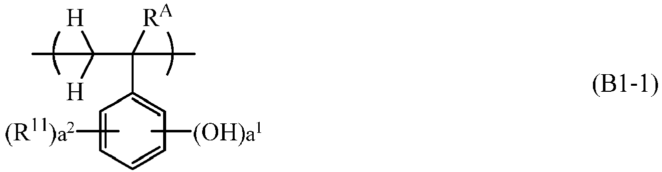 Herein R^{A}, R¹¹, a¹, and a² are as defined above.

Preferred examples of the repeat unit (B 1) include units derived from 3-hydroxystyrene, 4-hydroxystyrene, 5-hydroxy-2-vinylnaphthalene, and 6-hydroxy-2-vinylnaphthalene. Units of the formulae shown below are more preferred. 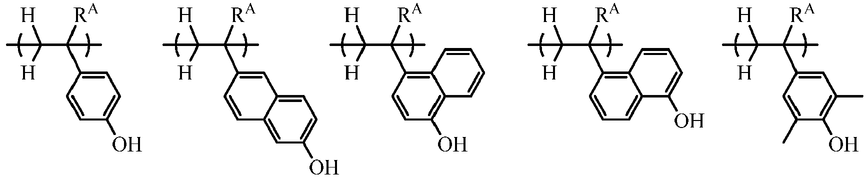 Herein R^{A} is as defined above.

In the embodiment wherein t¹=1, that is, the unit has an ester bond as the linker, preferred examples of the repeat unit (B 1) are shown below, but not limited thereto. 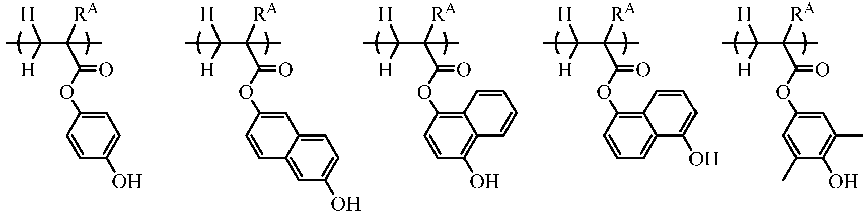 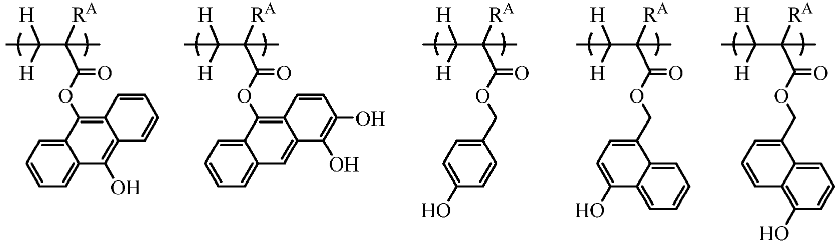 Herein R^{A} is as defined above.

The repeat units (B2) are preferably repeat units having the following formula (B2-1), which are also referred to as repeat units (B2-1), hereinafter. 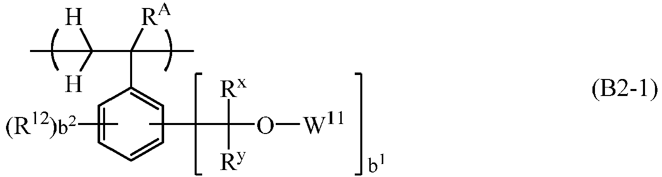 Herein R^{A}, R¹², R^{x}, R^{y}, W¹¹, b¹, and b² are as defined above.

Preferred examples of the repeat unit (B2) are shown below, but not limited thereto.

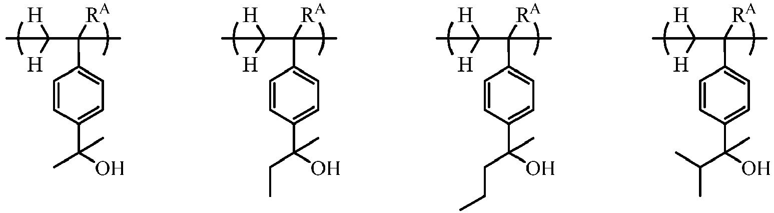

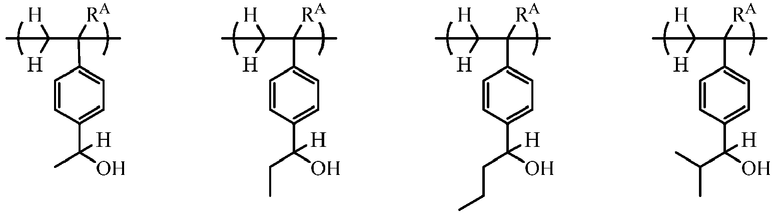

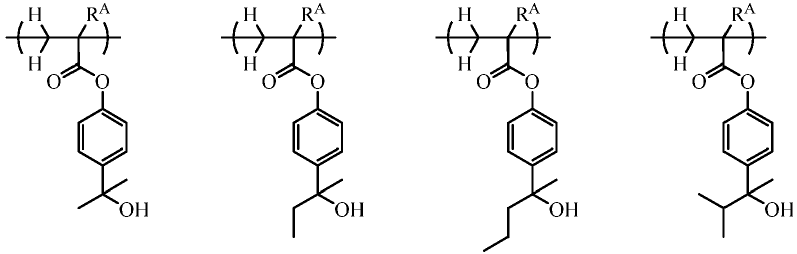

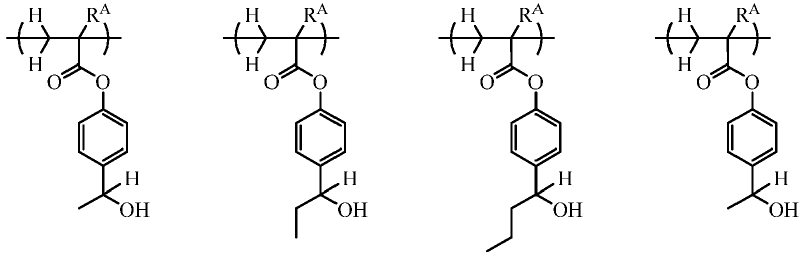

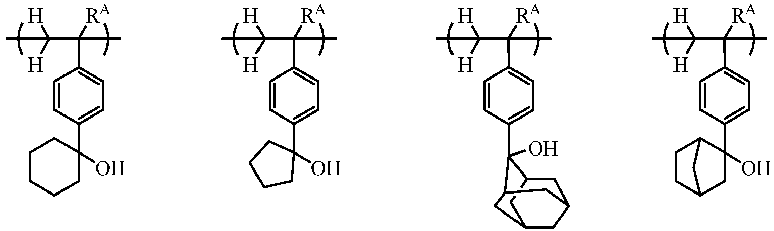

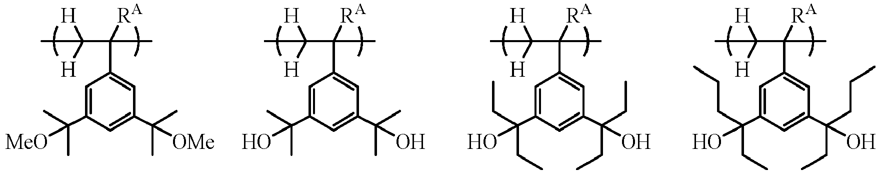

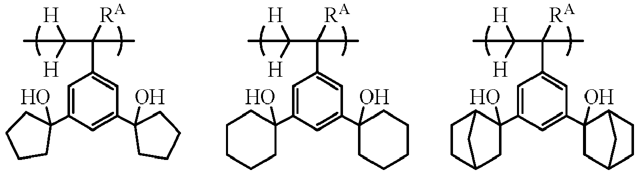

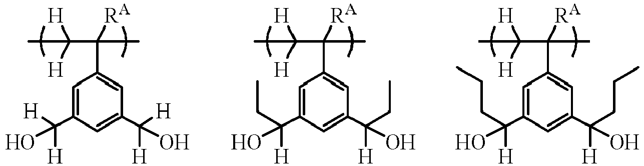

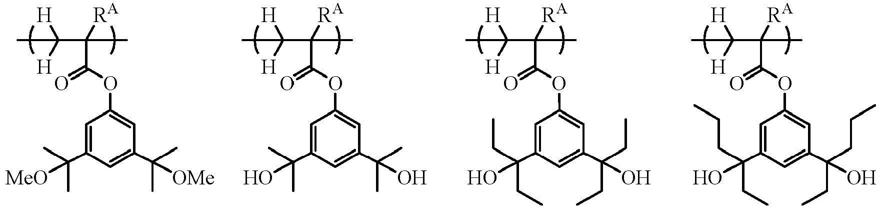

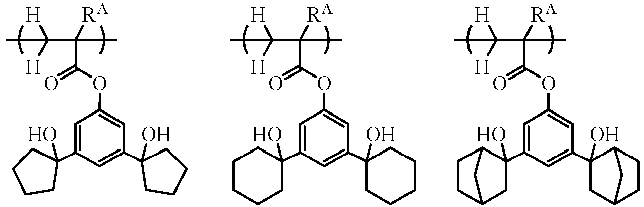

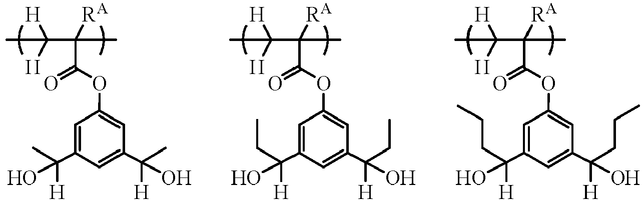

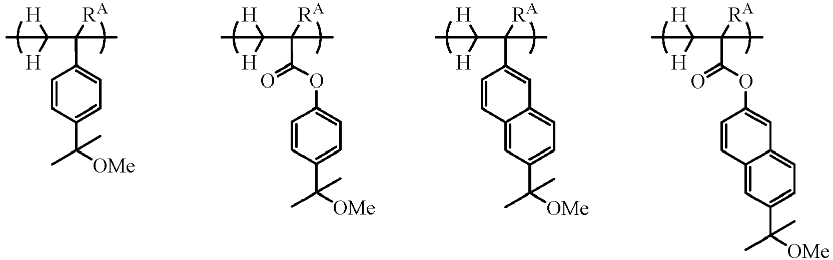

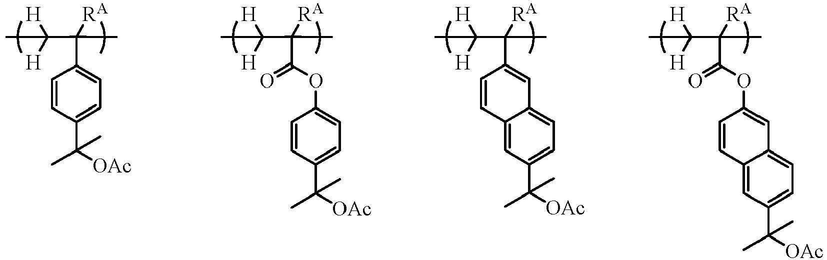

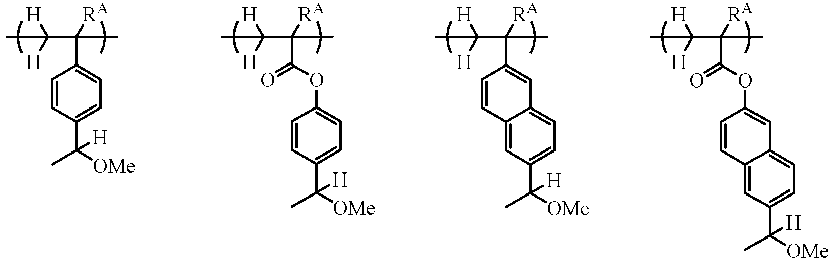

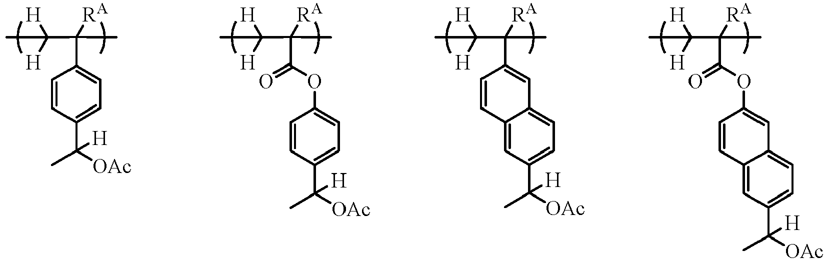

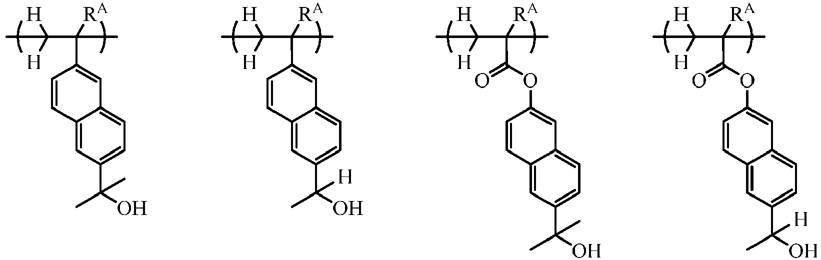

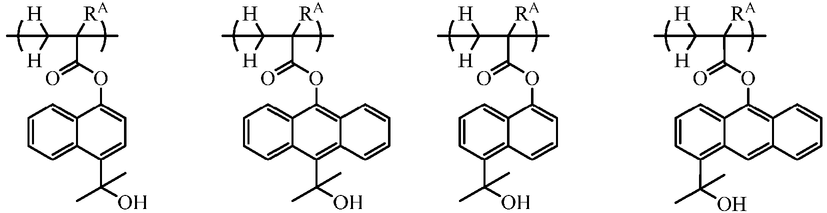

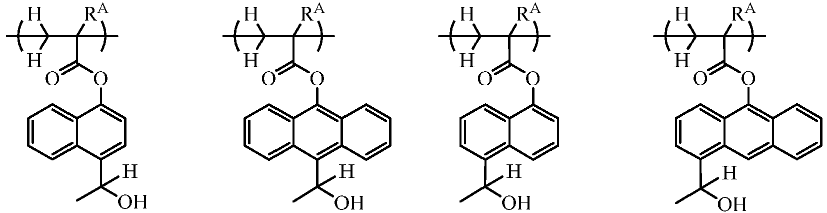

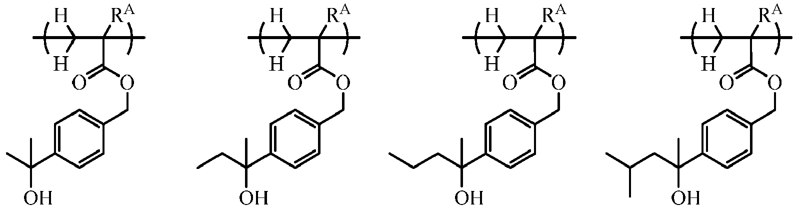

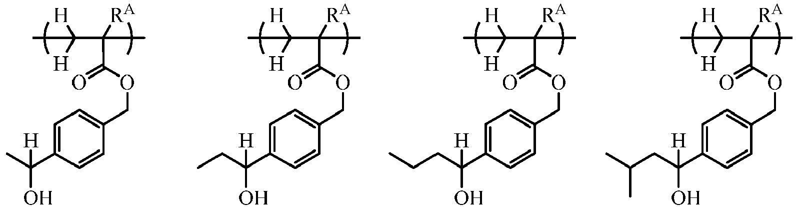

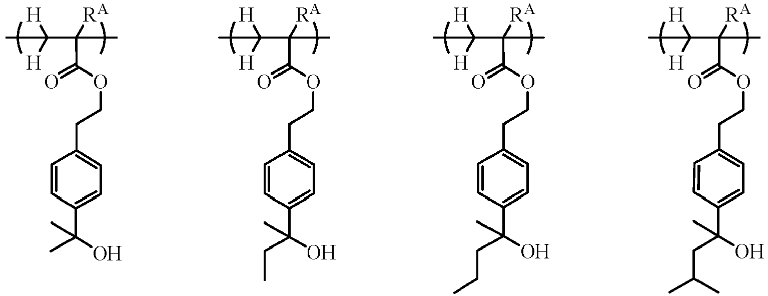

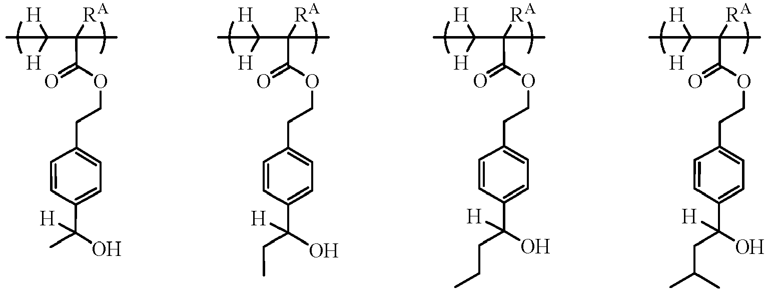

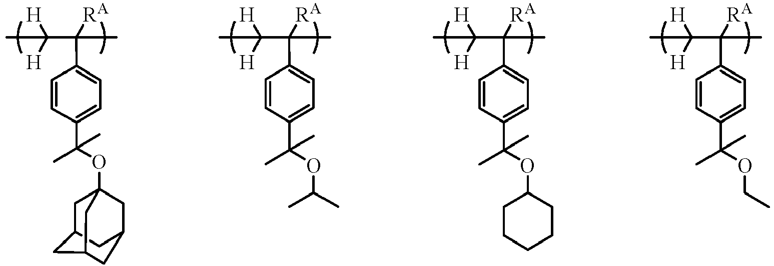

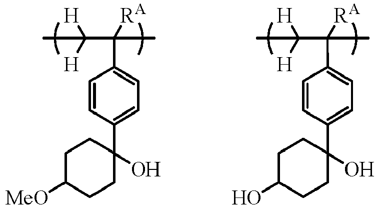

The content of repeat units is determined so as to establish a high contrast between the exposed region (which turns negative) and the unexposed region (which does not turn negative) upon exposure to high-energy radiation for the purpose of obtaining a high resolution. As to the content of repeat units (B 1), the lower limit is preferably 30 mol%, more preferably 40 mol%, even more preferably 50 mol%, and the upper limit is preferably 95 mol%, more preferably 90 mol%, even more preferably 80 mol%, based on the overall repeat units of the base polymer.

As to the content of repeat units (B2), the lower limit is preferably 5 mol%, more preferably 10 mol%, even more preferably 20 mol%, and the upper limit is preferably 70 mol%, more preferably 60 mol%, even more preferably 50 mol%, based on the overall repeat units of the base polymer.

Particularly when the base polymer contains repeat units (B 1-1) and (B2-1), the repeat units (B 1-1) contribute to a further improvement in etch resistance and improvements in adhesion to the substrate and dissolution in alkaline developer whereas the repeat units (B2-1) contribute to a more efficient progress of negative reaction and a further improvement in resolution.

For the purpose of enhancing etch resistance, the base polymer may further comprise repeat units of at least one type selected from repeat units having the formula (B3) and repeat units having the formula (B4). It is noted that these units are also referred to as repeat units (B3) and (B4), hereinafter.

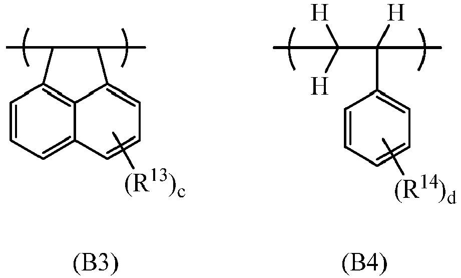

In formula (B3), R¹³ is each independently hydroxy, halogen, acetoxy, an optionally halogenated C₁-C₈ alkyl group, optionally halogenated C₁-C₈ saturated hydrocarbyloxy group, optionally halogenated C₂-C₈ saturated hydrocarbylcarbonyl group, or optionally halogenated C₂-C₈ saturated hydrocarbylcarbonyloxy group, with the proviso that R¹³ is not an acid labile group. In case of c ≥ 2, groups R¹³ may be the same or different.

In formula (B4), R¹⁴ is halogen, acetoxy, an optionally halogenated C₁-C₈ alkyl group, optionally halogenated C₁-C₈ saturated hydrocarbyloxy group, optionally halogenated C₂-C₈ saturated hydrocarbylcarbonyl group, or optionally halogenated C₂-C₈ saturated hydrocarbylcarbonyloxy group, with the proviso that R¹⁴ is not an acid labile group. In case of d ≥ 2, groups R¹⁴ may be the same or different.

In formulae (B3) and (B4), c and d are each independently an integer of 0 to 4.

When constituent units of at least one type selected from repeat units (B3) and (B4) are included, not only the aromatic ring inherently possesses etch resistance, but the ring structure incorporated in the main chain also exerts the effect of improving etch resistance and resistance to EB which is irradiated for pattern inspection.

Of the repeat units (B3) and (B4), units of one type or a mixture of two or more types may be used. The content of repeat units (B3) and/or (B4) is determined so as to achieve the effect of improving etch resistance, and its lower limit is preferably 2 mol%, more preferably 5 mol% and its upper limit is preferably 30 mol%, more preferably 20 mol%, based on the overall repeat units of the base polymer.

The base polymer may further comprise repeat units of at least one type selected from repeat units having the formula (B5), repeat units having the formula (B6), and repeat units having the formula (B7). It is noted that these units are also referred to as repeat units (B5), (B6) and (B7), hereinafter.

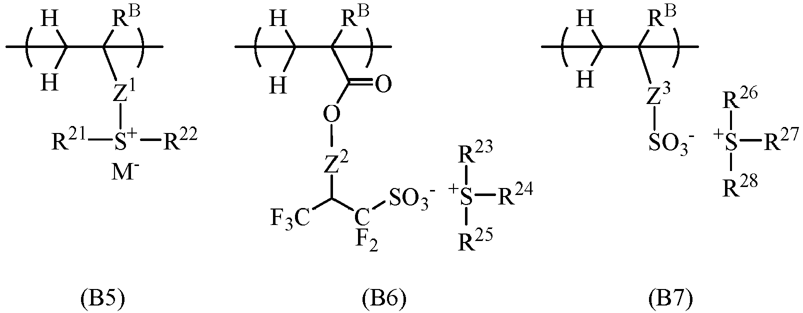

In formulae (B5) to (B7), R^{B} is each independently hydrogen or methyl. Z¹ is a single bond, a C₁-C₆ aliphatic hydrocarbylene group, phenylene group, naphthylene group, or C₇-C₁₈ group obtained by combining the foregoing, or -O-Z¹¹-, -C(=O)-O-Z¹¹- or -C(=O)-NH-Z¹¹-, wherein Z¹¹ is a C₁-C₆ aliphatic hydrocarbylene group, phenylene group, naphthylene group, or C₇-C₁₈ group obtained by combining the foregoing, which may contain a carbonyl moiety, ester bond, ether bond or hydroxy moiety. Z² is a single bond or -Z²¹-C(=O)-O-, wherein Z²¹ is a C₁-C₂₀ hydrocarbylene group which may contain a heteroatom. Z³ is a single bond, methylene, ethylene, phenylene, fluorinated phenylene, trifluoromethyl-substituted phenylene group, -O-Z³¹-, -C(=O)-O-Z³¹-, or -C(=O)-NH-Z³¹-, wherein Z³¹ is a C₁-C₁₀ aliphatic hydrocarbylene group, phenylene group, fluorinated phenylene group, trifluoromethyl-substituted phenylene group, or C₇-C₂₀ group obtained by combining the foregoing, which may contain a carbonyl moiety, ester bond, ether bond or hydroxy moiety.

In formula (B6), when Z² is -Z²¹-C(=O)-O-, Z²¹ is an optionally heteroatom-containing C₁-C₂₀ hydrocarbylene group, examples of which are shown below, but not limited thereto.

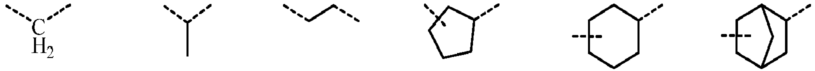

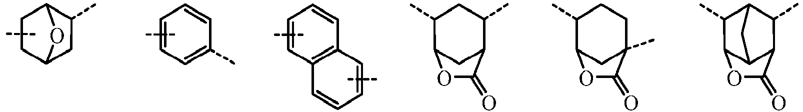

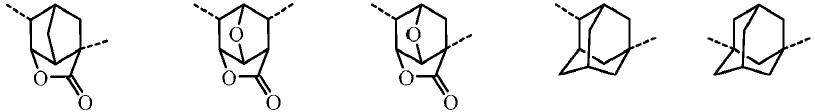

In formulae (B5) to (B7), R²¹ to R²⁸ are each independently halogen or a C₁-C₂₀ hydrocarbyl group which may contain a heteroatom. A pair of R²¹ and R²², R²³ and R²⁴, or R²⁶ and R²⁷ may bond together to form a ring with the sulfur atom to which they are attached. Examples of the optionally heteroatom-containing hydrocarbyl group are as exemplified above for R¹.

When R²¹ and R²², R²³ and R²⁴, or R²⁶ and R²⁷ bond together to form a ring with the sulfur atom to which they are attached, examples of the ring are shown below, but not limited thereto.

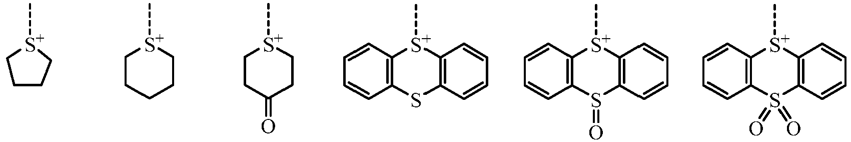

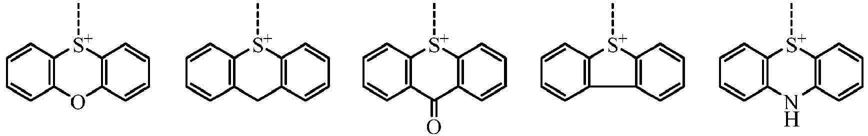

Exemplary structures of the sulfonium cation in formulae (B6) and (B7) are shown below, but not limited thereto.

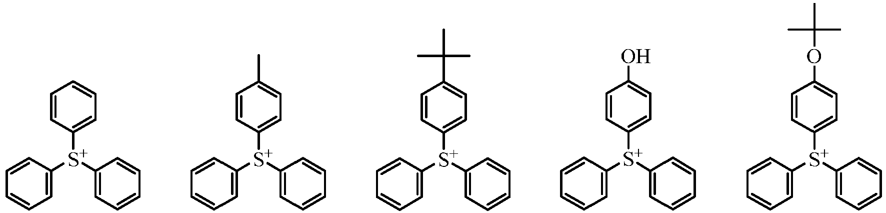

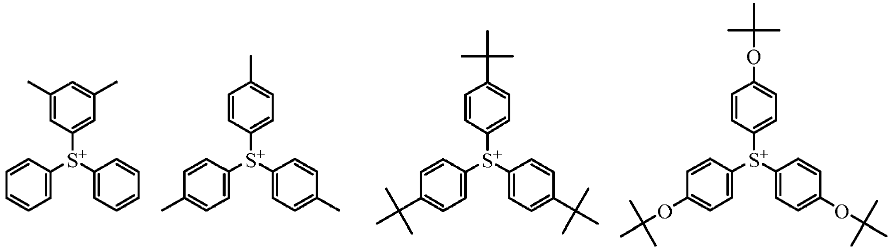

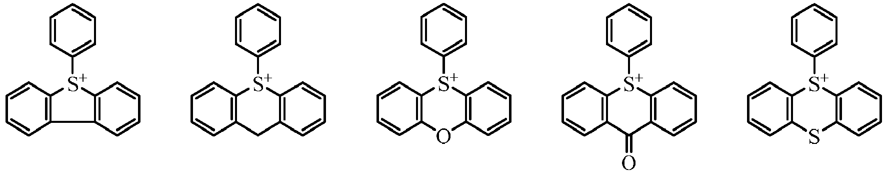

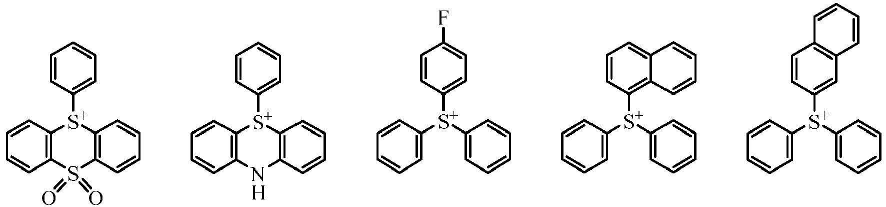

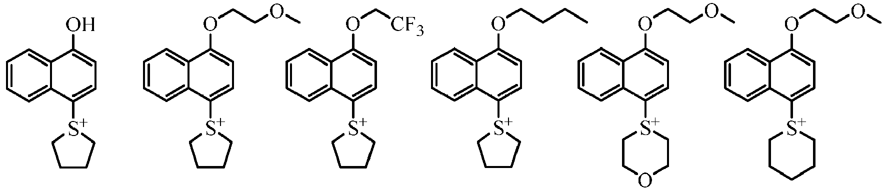

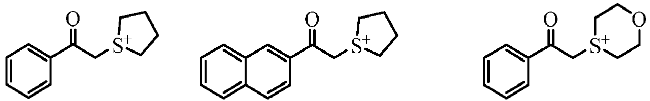

In formula (B5), M⁻ is a non-nucleophilic counter ion.

The repeat units (B5) to (B7) are units adapted to generate an acid upon exposure to high-energy radiation. The inclusion of these units in the polymer is effective for suppressing acid diffusion adequately and forming a pattern with reduced LER or LWR.
The inclusion of these units in the polymer is also effective for preventing the acid from volatilizing from the exposed region to deposit on the unexposed region again during bake in vacuum for thereby reducing LER or LWR, and for suppressing undesired negative reaction in the unexposed region for thereby reducing defects. When the base polymer contains repeat units (B5) to (B7), their content is preferably 0.5 to 20 mol% based on the overall repeat units.

Further, the base polymer may contain (meth)acrylate units protected with a commonly used acid labile group and/or (meth)acrylate units having an adhesive group such as lactone structure. The inclusion of these units allows for fine adjustment of properties of a resist film, but is not critical.

Examples of the (meth)acrylate unit having an adhesive group include units having the following formulae (B8) to (B 10). These units, which do not exhibit acidity, may be used as auxiliary units for promoting adhesion to the substrate and/or for adjusting solubility.

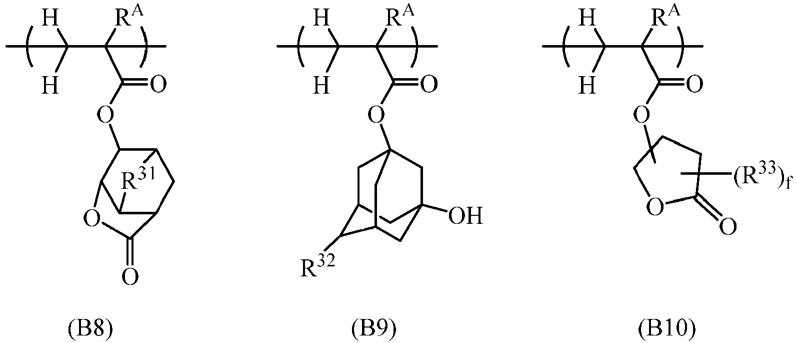

In formulae (B8) to (B 10), R^{A} is as defined above, R³¹ is -O- or -CH₂-, R³² is hydrogen or hydroxy, R¹³ is a C₁-C₄ saturated hydrocarbyl group, and f is an integer of 0 to 3.

The content of such repeat units is preferably 0 to 30 mol%, more preferably 0 to 20 mol% based on the overall repeat units of the base polymer.

Among others, a base polymer containing repeat units having formula (B 1-2), repeat units having formula (B2-2), and repeat units having formula (B6) as shown below is preferred. 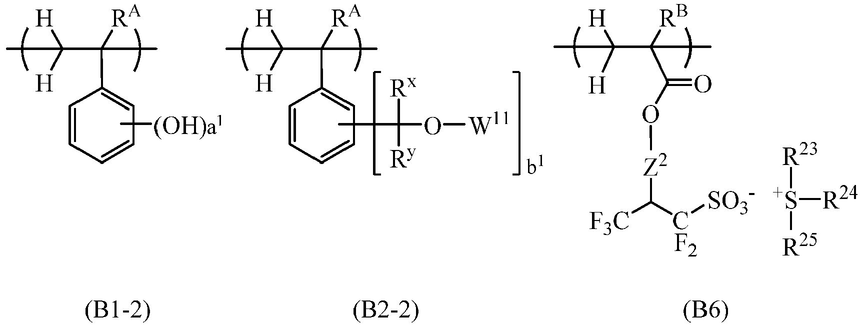 Herein R^{A}, R^{B}, Z², R²³ to R²¹, R^{x}, R^{y}, W¹¹, a¹, and b¹ are as defined above.

In a further embodiment, the base polymer is a mixture of a base polymer free of repeat units (B5) to (B7) and a base polymer containing any one of repeat units (B5) to (B7). Preferred is a mixture of 80 parts by weight of the base polymer containing any one of repeat units (B5) to (B7) and 2 to 5,000 parts by weight, especially 10 to 1,000 parts by weight of the base polymer free of repeat units (B5) to (B7).

Reference is now made to the use of the chemically amplified negative resist composition in the fabrication of photomasks. The lithography of the advanced generation employs a coating film having a thickness of up to 150 nm, preferably up to 100 nm. Since an intense development process is often employed to minimize defects resulting from resist residues, the base polymer should preferably have a dissolution rate in alkaline developer (typically 2.38 wt% tetramethylammonium hydroxide (TMAH) aqueous solution) of up to 80 nm/sec, more preferably up to 50 nm/sec in order to form a small size pattern. When the chemically amplified negative resist composition is used in the EUV lithography process for fabricating an LSI chip from a wafer, for example, the coating film often has a thickness of up to 100 nm, in view of the necessity of patterning narrow lines of 50 nm or less. In consideration of the risk that the pattern of such thin film can be degraded by development, the polymer preferably has a dissolution rate of up to 80 nm/sec, more preferably up to 50 nm/sec. On the other hand, a thick film having a thickness of 200 nm or greater is often employed in the KrF lithography process although the film thickness depends on a particular purpose. In this case, the polymer is preferably designed to a dissolution rate of at least 90 nm/sec.

The polymer may be synthesized by copolymerizing monomers optionally protected with a protective group, and optionally effecting deprotection reaction. The copolymerization reaction is preferably radical or anionic polymerization, but not limited thereto. For copolymerization, reference should be made to WO 2006/121096, JP-A 2008-102383, JP-A 2008-304590, and JP-A 2004-115630.

The base polymer should preferably have a weight average molecular weight (Mw) in the range of 1,000 to 500,000, and more preferably 2,000 to 20,000, as measured by GPC versus polystyrene standards. As is well known in the art, a Mw of at least 1,000 eliminates the risk of rounding of pattern top which invites a lowering of resolution and degradation of LER or LWR. A Mw of up to 50,000 eliminates the risk of LER or LWR increasing when a pattern having a line width of up to 100 nm is formed.

The base polymer should preferably have a narrow dispersity (Mw/Mn) of 1.0 to 2.0, especially 1.0 to 1.8. A Mw/Mn in the range eliminates the risks that foreign particles are left on the pattern after development and the pattern profile is aggravated.

### Other components

In addition to the crosslinker and base polymer, the chemically amplified negative resist composition of the invention may further contain other components such as an organic solvent, acid generator, quencher, fluorinated polymer, and surfactant, if necessary and in any suitable combination. The chemically amplified negative resist composition thus constructed has the advantages that the dissolution in alkaline developer is suppressed and a pattern of good profile with minimal top loss is formed, as compared with negative patterns formed from prior art negative resist compositions. In addition, the resist film has a high dissolution contrast, resolution, exposure latitude, and process adaptability, and provides a good pattern profile after exposure, and minimal proximity bias because of restrained acid diffusion. By virtue of these advantages, the composition is fully useful in commercial application and suited as a pattern-forming material for the fabrication of VLSIs.

### Organic solvent

The organic solvent used herein is not particularly limited as long as the foregoing and other components are soluble therein. Examples of the organic solvent are described in JP-A 2008-111103, paragraphs [0144]-[0145] (USP 7,537,880). Exemplary solvents include ketones such as cyclohexanone, cyclopentanone, methyl-2-n-pentyl ketone and 2-heptanone; alcohols such as 3-methoxybutanol, 3-methyl-3-methoxybutanol, 1-methoxy-2-propanol, 1-ethoxy-2-propanol, and diacetone alcohol (DAA); ethers such as propylene glycol monomethyl ether (PGME), ethylene glycol monomethyl ether, propylene glycol monoethyl ether, ethylene glycol monoethyl ether, propylene glycol dimethyl ether, and diethylene glycol dimethyl ether; esters such as propylene glycol monomethyl ether acetate (PGMEA), propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate; and lactones such as γ-butyrolactone, which may be used alone or in admixture.

Of these solvents, 1-ethoxy-2-propanol, PGMEA, PGME, ethyl lactate, cyclohexanone, γ-butyrolactone, and mixtures thereof are preferred because many components are fully soluble therein.

The organic solvent is preferably added in an amount of 200 to 12,000 parts, and more preferably 400 to 10,000 parts by weight per 80 parts by weight of the base polymer.

### Acid generator

The acid generator, also referred to as acid generator of addition type, is typically a compound (PAG) capable of generating an acid in response to actinic ray or radiation. The PAG used herein may be any compound capable of generating an acid upon exposure to high-energy radiation. Suitable PAGs include sulfonium salts, iodonium salts, sulfonyldiazomethane, N-sulfonyloxyimide, and oxime-O-sulfonate acid generators, which may be used alone or in admixture of two or more.

Suitable PAGs include nonafluorobutane sulfonate, partially fluorinated sulfonates described in JP-A 2012-189977, paragraphs [0247]-[0251], partially fluorinated sulfonates described in JP-A 2013-101271, paragraphs [0261]-[0265], and those described in JP-A 2008-111103, paragraphs [0122]-[0142] and JP-A 2010-215608, paragraphs [0080]-[0081]. Among others, arylsulfonate and alkanesulfonate type PAGs are preferred because they generate acids having an appropriate strength to promote reaction of the crosslinker with the base polymer.

The PAG capable of generating an acid having a pKa value in the range of -3.0 to 1.5, more preferably -1.0 to 1.5 is preferred because the effect of improving LER or LWR by combining the generated acid with an onium salt (to be described later) to induce exchange reaction is achievable. The preferred acid generators are compounds having a sulfonium anion of the structure shown below. Notably the cation that pairs with the anion is as exemplified for the sulfonium cation in formulae (B6) and (B7)

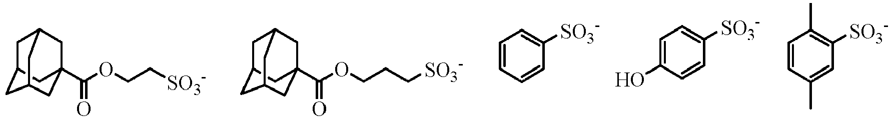

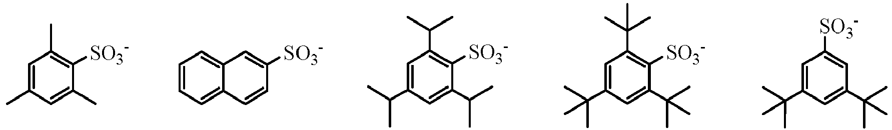

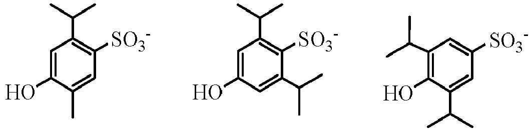

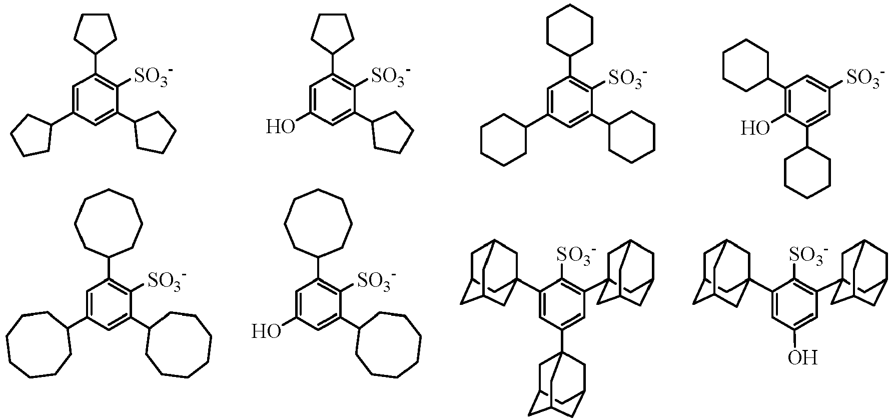

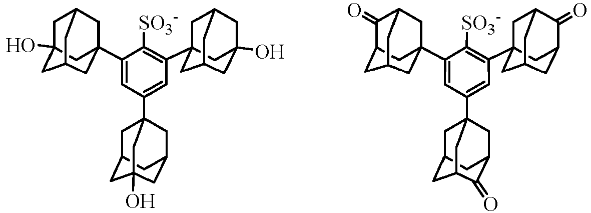

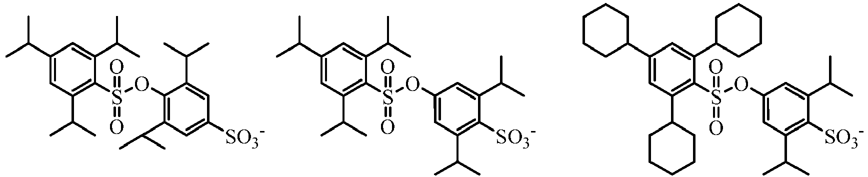

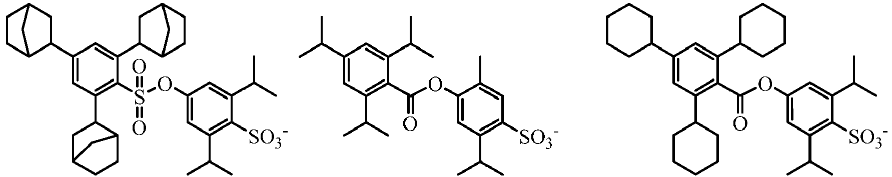

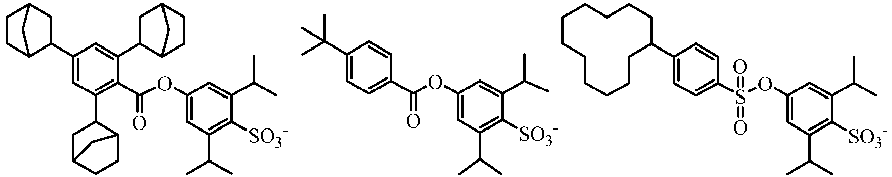

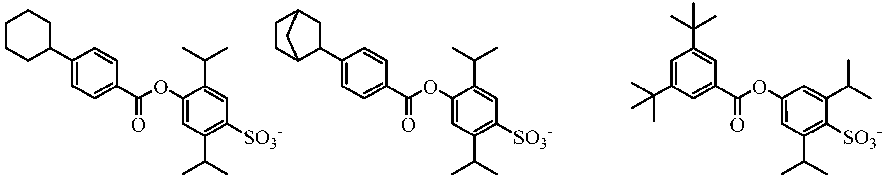

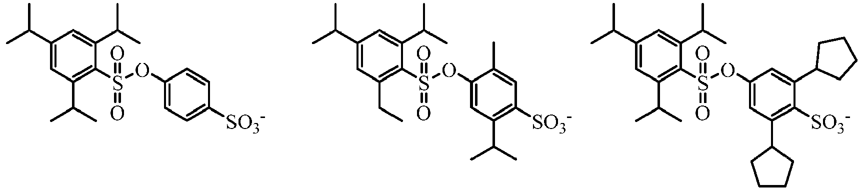

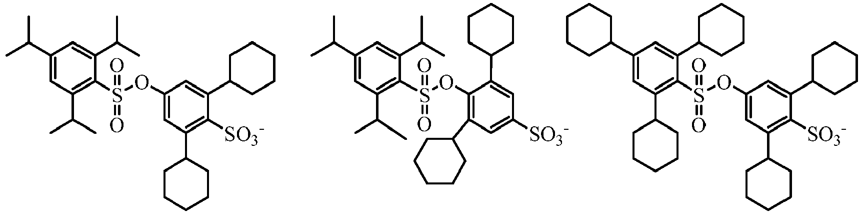

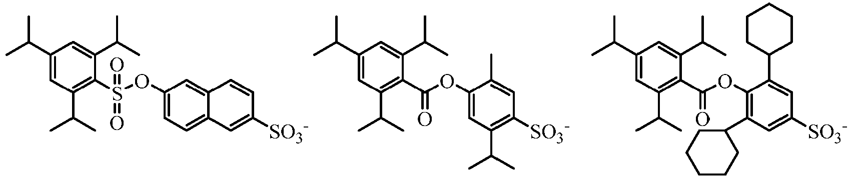

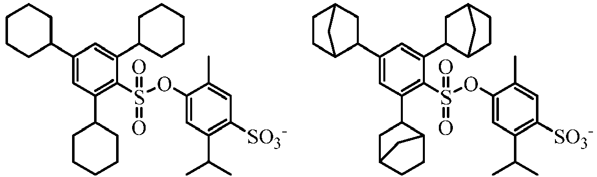

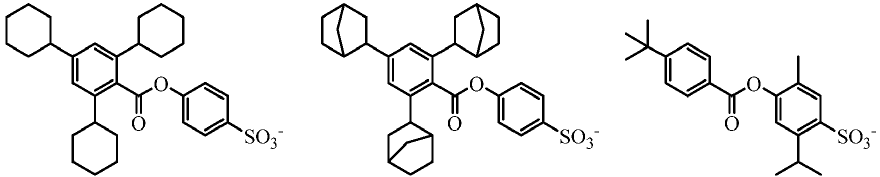

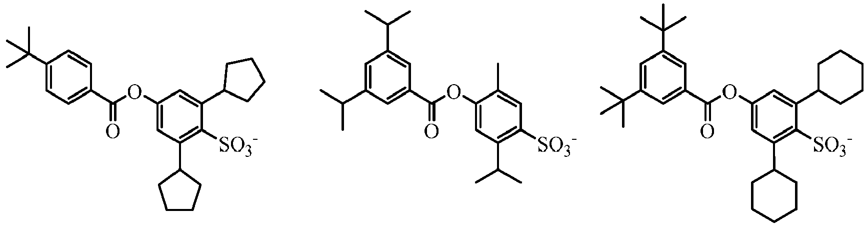

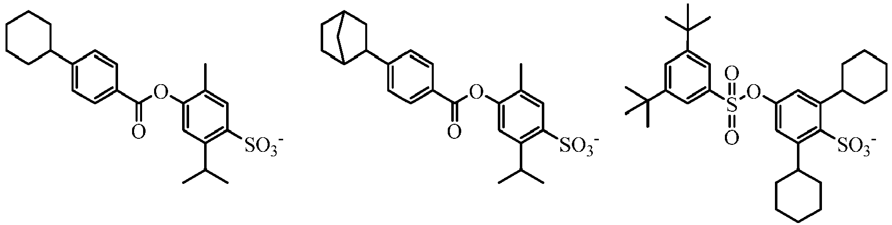

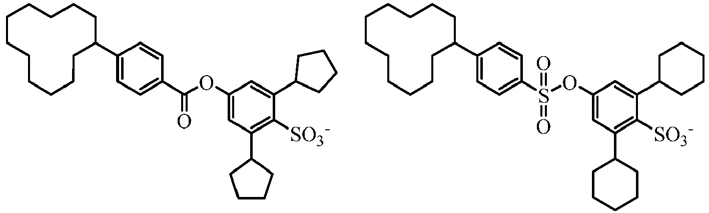

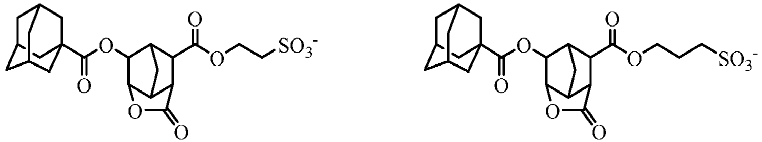

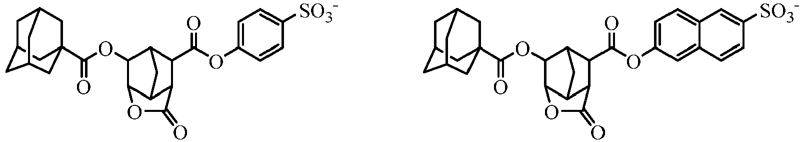

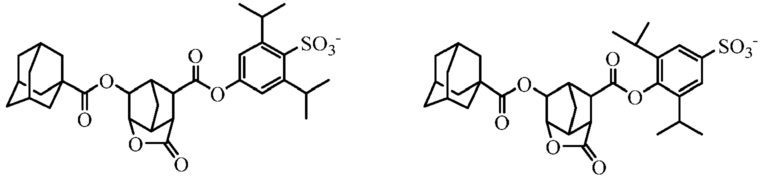

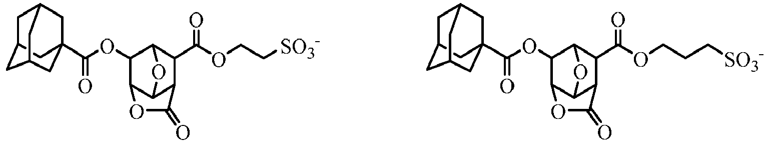

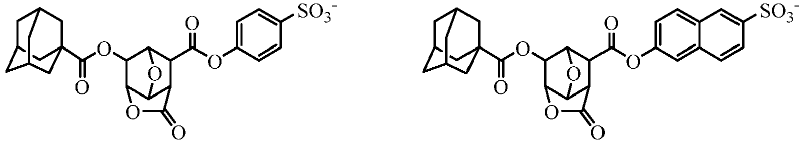

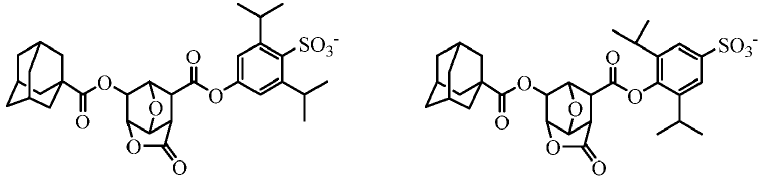

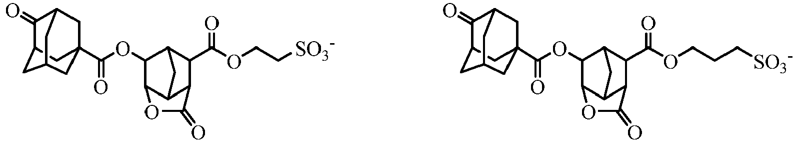

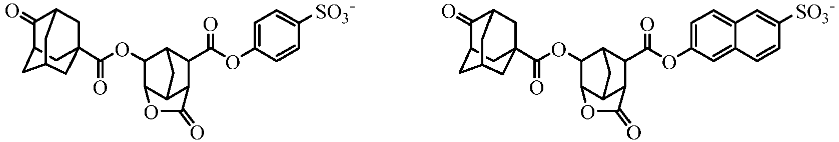

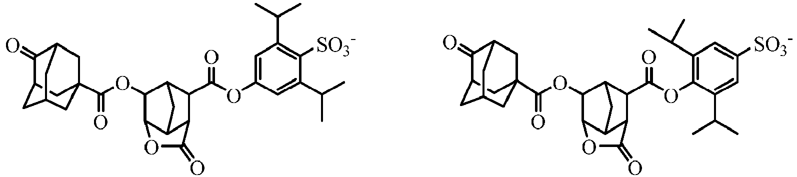

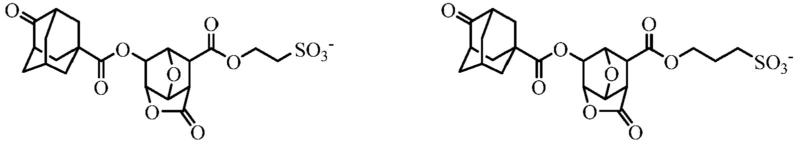

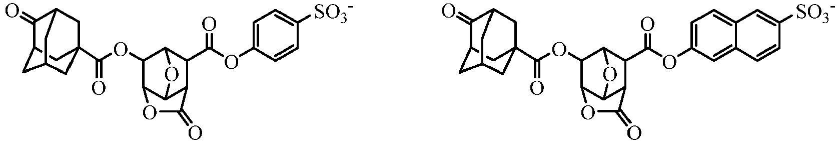

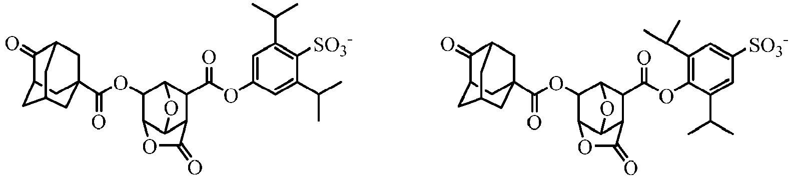

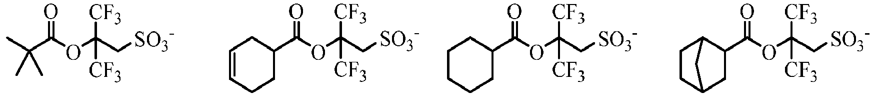

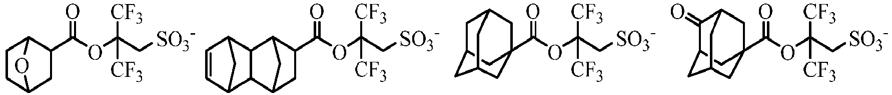

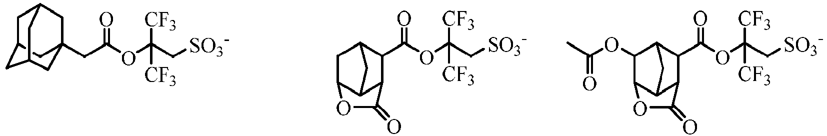

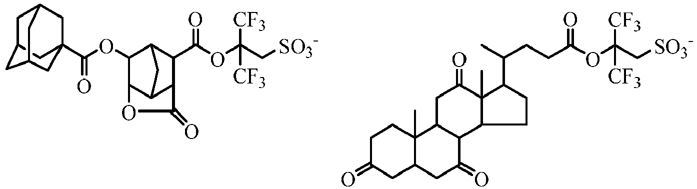

An appropriate amount of the acid generator of addition type used is 2 to 20 parts, more preferably 5 to 15 parts by weight per 80 parts by weight of the base polymer. The acid generator may be used alone or in admixture. Where the base polymer contains repeat units (B5) to (B7) (that is, in the case of polymer-bound acid generator), the acid generator of addition type may be omitted.

### Quencher

The resist composition may contain a quencher which is typically selected from conventional basic compounds. Conventional basic compounds include primary, secondary, and tertiary aliphatic amines, mixed amines, aromatic amines, heterocyclic amines, nitrogen-containing compounds with carboxy group, nitrogen-containing compounds with sulfonyl group, nitrogen-containing compounds with hydroxy group, nitrogen-containing compounds with hydroxyphenyl group, alcoholic nitrogen-containing compounds, amide derivatives, imide derivatives, and carbamate derivatives. Also included are primary, secondary, and tertiary amine compounds, specifically amine compounds having a hydroxy group, ether bond, ester bond, lactone ring, cyano group, or sulfonic ester bond as described in JP-A 2008-111103, paragraphs [0146]-[0164], and compounds having a carbamate group as described in JP 3790649. Addition of a basic compound is effective for further suppressing the diffusion rate of acid in the resist film or correcting the pattern profile.

Onium salts such as sulfonium, iodonium and ammonium salts of carboxylic acids which are not fluorinated at α-position as described in USP 8,795,942 (JP-A 2008-158339) may also be used as the quencher. While an α-fluorinated sulfonic acid, imide acid, and methide acid are necessary to deprotect the acid labile group, an α-non-fluorinated carboxylic acid is released by salt exchange with an α-non-fluorinated carboxylic onium salt. The α-non-fluorinated carboxylic acid functions as a quencher because it induces no or little deprotection reaction.

Examples of the onium salt of α-non-fluorinated carboxylic acid include compounds having the formula (C1).

R¹⁰¹-CO₂⁻ Mq⁺ (C1)

In formula (C1), R¹⁰¹ is hydrogen or a C₁-C₄₀ hydrocarbyl group which may contain a heteroatom, exclusive of the hydrocarbyl group in which the hydrogen bonded to the carbon atom at α-position of the carboxy group is substituted by fluorine or fluoroalkyl.

The hydrocarbyl group may be saturated or unsaturated and straight, branched or cyclic. Examples thereof include alkyl groups such as methyl, ethyl, propyl, isopropyl, n-butyl, sec-butyl, tert-butyl, n-pentyl, tert-pentyl, n-hexyl, n-octyl, 2-ethylhexyl, n-nonyl, n-decyl; cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decanyl, adamantyl, adamantylmethyl; alkenyl groups such as vinyl, allyl, propenyl, butenyl, hexenyl; cyclic unsaturated aliphatic hydrocarbyl groups such as cyclohexenyl; aryl groups such as phenyl, naphthyl, alkylphenyl groups (e.g., 2-methylphenyl, 3-methylphenyl, 4-methylphenyl, 4-ethylphenyl, 4-tert-butylphenyl, 4-n-butylphenyl), dialkylphenyl groups (e.g., 2,4-dimethylphenyl and 2,4,6-triisopropylphenyl), alkylnaphthyl groups (e.g., methylnaphthyl and ethylnaphthyl); and aralkyl groups such as benzyl, 1-phenylethyl and 2-phenylethyl.

In these groups, some hydrogen may be substituted by a moiety containing a heteroatom such as oxygen, sulfur, nitrogen or halogen, and some carbon may be replaced by a moiety containing a heteroatom such as oxygen, sulfur or nitrogen, so that the group may contain a hydroxy moiety, cyano moiety, carbonyl moiety, ether bond, thioether bond, ester bond, sulfonic ester bond, carbonate bond, lactone ring, sultone ring, carboxylic anhydride, or haloalkyl moiety. Examples of the heteroatom-containing hydrocarbyl group include heteroaryl groups such as thienyl; alkoxyphenyl groups such as 4-hydroxyphenyl, 4-methoxyphenyl, 3-methoxyphenyl, 2-methoxyphenyl, 4-ethoxyphenyl, 4-tert-butoxyphenyl, 3-tert-butoxyphenyl; alkoxynaphthyl groups such as methoxynaphthyl, ethoxynaphthyl, n-propoxynaphthyl, n-butoxynaphthyl; dialkoxynaphthyl groups such as dimethoxynaphthyl, diethoxynaphthyl; and aryloxoalkyl groups, typically 2-aryl-2-oxoethyl groups such as 2-phenyl-2-oxoethyl, 2-(1-naphthyl)-2-oxoethyl, 2-(2-naphthyl)-2-oxoethyl.

In formula (C1), Mq⁺ is an onium cation. The preferred onium cations include sulfonium, iodonium and ammonium cations, with the sulfonium and iodonium cations being more preferred.

A sulfonium salt of iodized benzene ring-containing carboxylic acid having the formula (C2) is also useful as the quencher.

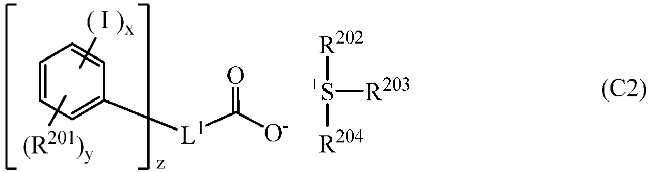

In formula (C2), R²⁰¹ is hydroxy, fluorine, chlorine, bromine, amino, nitro, cyano, or a C₁-C₆ saturated hydrocarbyl group, C₁-C₆ saturated hydrocarbyloxy group, C₂-C₆ saturated hydrocarbylcarbonyloxy group or C₁-C₄ saturated hydrocarbylsulfonyloxy group, in which some or all hydrogen may be substituted by halogen, or -N(R^{201A})-C(=O)-R^{201B}, or -N(R^{201A})-C(=O)-O-R^{201B}, wherein R^{201A} is hydrogen or a C₁-C₆ saturated hydrocarbyl group group and R^{201B} is a C₁-C₆ saturated hydrocarbyl group or C₂-C₈ unsaturated aliphatic hydrocarbyl group.

In formula (C2), x is an integer of 1 to 5, y is an integer of 0 to 3, and z is an integer of 1 to 3. L¹ is a single bond, or a C₁-C₂₀ (z+1)-valent linking group which may contain an ether bond, carbonyl, ester bond, amide bond, sultone ring, lactam ring, carbonate, halogen, hydroxy or carboxy moiety. The saturated hydrocarbyl group, saturated hydrocarbyloxy group, saturated hydrocarbylcarbonyloxy group and saturated hydrocarbylsulfonyloxy groups may be straight, branched or cyclic. When y and/or z is 2 or more, groups R²⁰¹ may be the same or different.

In formula (C2), R²⁰², R²⁰³ and R²⁰⁴ are each independently halogen or a C₁-C₂₀ hydrocarbyl group which may contain a heteroatom. The hydrocarbyl group may be saturated or unsaturated and straight, branched or cyclic. Examples thereof include C₁-C₂₀ alkyl, C₂-C₂₀ alkenyl, C₆-C₂₀ aryl, and C₇-C₂₀ aralkyl groups. In these groups, some or all hydrogen may be substituted by hydroxy, carboxy, halogen, oxo, cyano, nitro, sultone, sulfone, or sulfonium salt-containing moiety, or some carbon may be replaced by an ether bond, ester bond, carbonyl, amide bond, carbonate or sulfonic ester bond. A pair of R²⁰² and R²⁰³ may bond together to form a ring with the sulfur atom to which they are attached.

Examples of the compound having formula (C2) include those described in JP-A 2017-219836. The compound is highly absorptive and has a high sensitizing effect and acid diffusion controlling effect.

Betaine type compounds of weak acids are also useful as the quencher. Their examples are shown below, but not limited thereto.

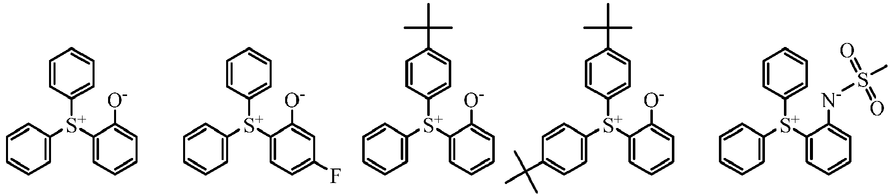

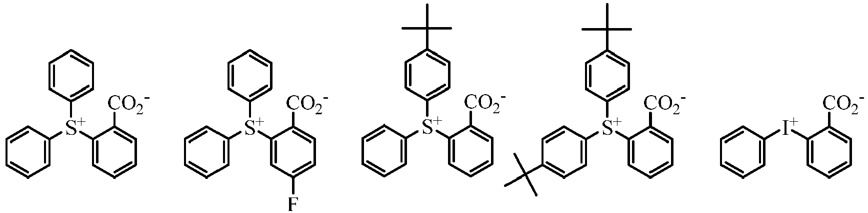

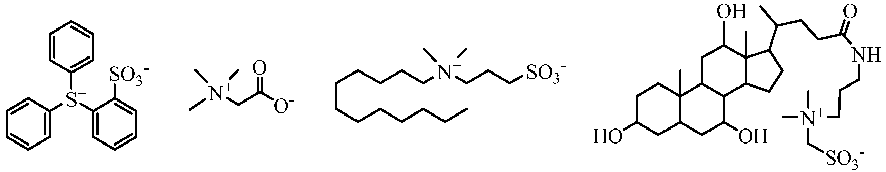

Also useful are quenchers of polymer type as described in USP 7,598,016 (JP-A 2008-239918). The polymeric quencher segregates at the resist surface after coating and thus enhances the rectangularity of resist pattern. When a protective film is applied as is often the case in the immersion lithography, the polymeric quencher is also effective for preventing a film thickness loss of resist pattern or rounding of pattern top.

The quencher is preferably added in an amount of 0 to 40 parts, more preferably 0 to 30 parts by weight per 80 parts by weight of the base polymer.

### Fluorinated polymer

The resist composition may further comprise a fluorinated polymer comprising repeat units having the formula (D1) and repeat units of at least one type selected from repeat units having the formulae (D2), (D3), (D4), and (D5), for the purposes of enhancing contrast, preventing chemical flare of acid upon exposure to high-energy radiation, and suppressing unexpected unnecessary pattern degradation. Notably, repeat units having formulae (D1), (D2), (D3), (D4), and (D5) are simply referred to as repeat units (D1), (D2), (D3), (D4), and (D5), respectively. Since the fluorinated polymer also has a surface active function, it can prevent insoluble residues from re-depositing onto the substrate during the development step and is thus effective for preventing development defects.

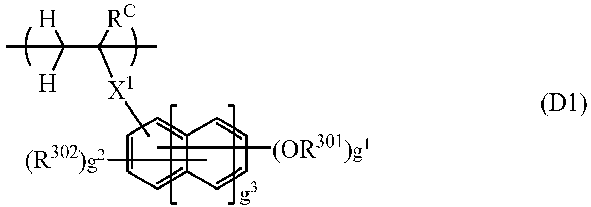

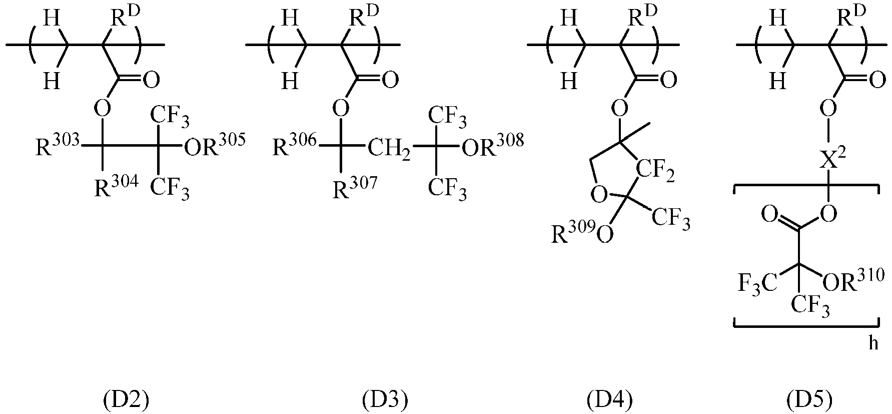

Herein R^{C} is each independently hydrogen or methyl. R^{D} is each independently hydrogen, fluorine, methyl or trifluoromethyl. R³⁰¹ is hydrogen or a C₁-C₅ straight or branched hydrocarbyl group in which a heteroatom-containing moiety may intervene in a carbon-carbon bond. R³⁰² is a C₁-C₅ straight or branched hydrocarbyl group in which a heteroatom-containing moiety may intervene in a carbon-carbon bond. R³⁰³, R³⁰⁴, R³⁰⁶ and R³⁰⁷ are each independently hydrogen or a C₁-C₁₀ saturated hydrocarbyl group. R³⁰⁵, R³⁰⁸, R³⁰⁹ and R³¹⁰ are each independently hydrogen, a C₁-C₁₅ hydrocarbyl group or fluorinated hydrocarbyl group, or an acid labile group, with the proviso that an ether or carbonyl moiety may intervene in a carbon-carbon bond in the hydrocarbyl groups or fluorinated hydrocarbyl groups represented by R³⁰⁵, R³⁰⁸, R³⁰⁹ and R³¹⁰. The subscript g¹ is an integer of 1 to 3, g² is an integer satisfying: 0 ≤ g² ≤ 5+2g³-g¹, g³ is 0 or 1, h is an integer of 1 to 3. X¹ is a single bond, -C(=O)-O- or -C(=O)-NH-. X² is a C₁-C₂₀ (h+1)-valent hydrocarbon group or fluorinated hydrocarbon group.

Suitable C₁-C₅ hydrocarbyl groups R³⁰¹ and R³⁰² include alkyl, alkenyl and alkynyl groups, with the alkyl groups being preferred. Suitable alkyl groups include methyl, ethyl, n-propyl, isopropyl, n-butyl, isobutyl, sec-butyl, and n-pentyl. In these groups, a moiety containing a heteroatom such as oxygen, sulfur or nitrogen may intervene in a carbon-carbon bond.

In formula (D1), -OR³⁰¹ is preferably a hydrophilic group. In this case, R³⁰¹ is preferably hydrogen or a C₁-C₅ alkyl group in which oxygen intervenes in a carbon-carbon bond.

Examples of the repeat unit (D1) are given below, but not limited thereto. Herein R^{C} is as defined above.

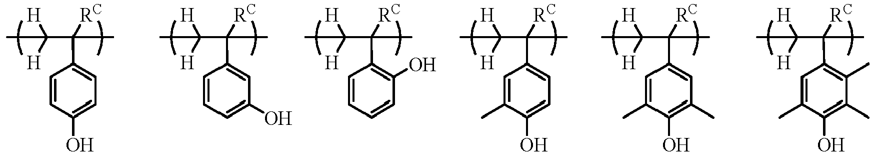

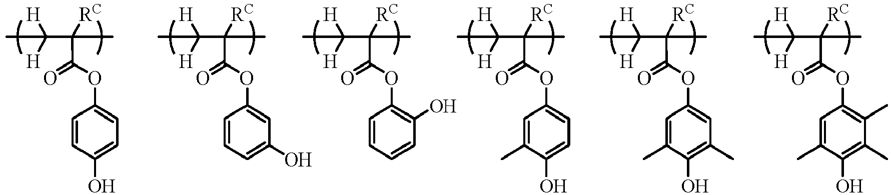

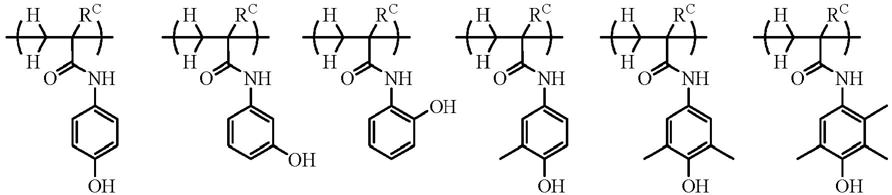

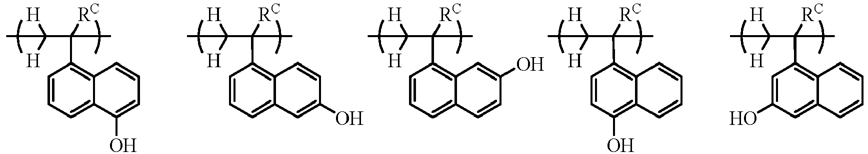

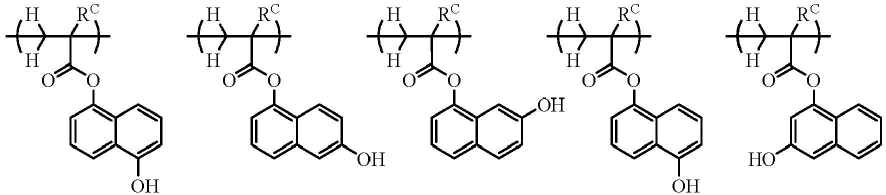

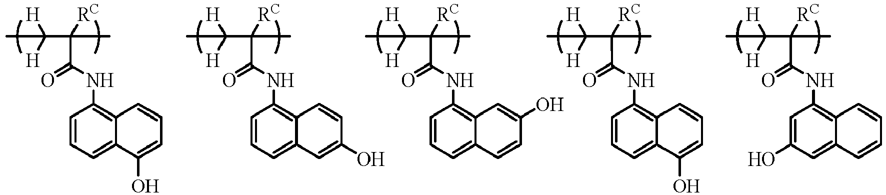

In formula (D1), X¹ is preferably -C(=O)-O- or -C(=O)-NH-. Also preferably R^{C} is methyl. The inclusion of carbonyl in X¹ enhances the ability to trap the acid originating from an anti-charging film. A polymer wherein R^{C} is methyl is a rigid polymer having a high glass transition temperature (Tg) which is effective for suppressing acid diffusion. As a result, the stability with time of a resist film is improved, and neither resolution nor pattern profile is degraded.

In formulae (D2) and (D3), examples of the C₁-C₁₀ saturated hydrocarbyl group represented by R³⁰³, R³⁰⁴, R³⁰⁶ and R³⁰⁷ include alkyl groups such as methyl, ethyl, n-propyl, isopropyl, n-butyl, isobutyl, sec-butyl, tert-butyl, n-pentyl, n-hexyl, n-heptyl, n-octyl, n-nonyl, n-decyl; and cyclic saturated hydrocarbyl groups such as cyclopropyl, cyclobutyl, cyclopentyl, cyclohexyl, adamantyl, and norbornyl. Inter alia, C₁-C₆ saturated hydrocarbyl groups are preferred.

In formulae (D2) to (D5), examples of the C₁-C₁₅ hydrocarbyl group represented by R³⁰⁵, R³⁰⁸, R³⁰⁹ and R³¹⁰ include alkyl, alkenyl and alkynyl groups, with the alkyl groups being preferred. Suitable alkyl groups include n-undecyl, n-dodecyl, tridecyl, tetradecyl and pentadecyl as well as those exemplified above. The fluorinated hydrocarbyl groups correspond to the foregoing hydrocarbyl groups in which some or all carbon-bonded hydrogen atoms are substituted by fluorine atoms.

Examples of the C₁-C₂₀ (h+1)-valent hydrocarbon group or fluorinated hydrocarbon group X² include the foregoing hydrocarbyl groups and fluorinated hydrocarbyl groups, with number (h) of hydrogen atoms being eliminated.

Examples of the repeat units (D2) to (D5) are given below, but not limited thereto. Herein R^{D} is as defined above.

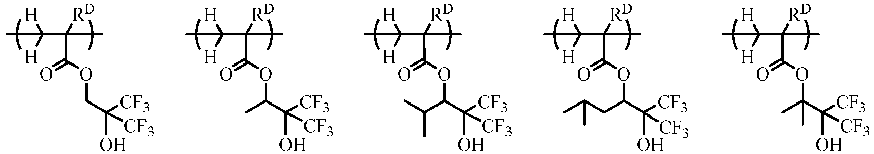

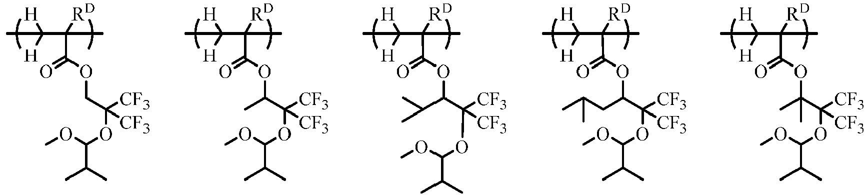

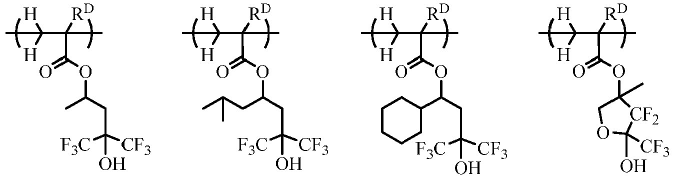

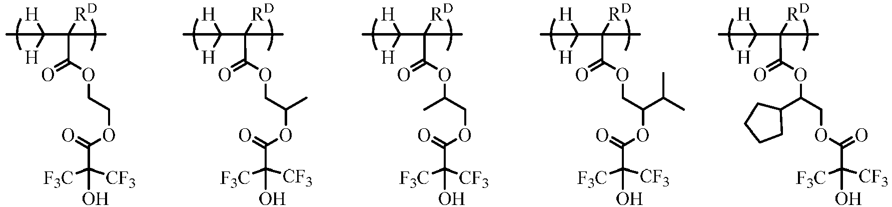

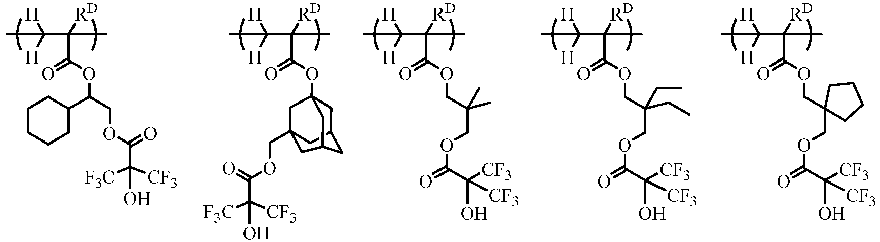

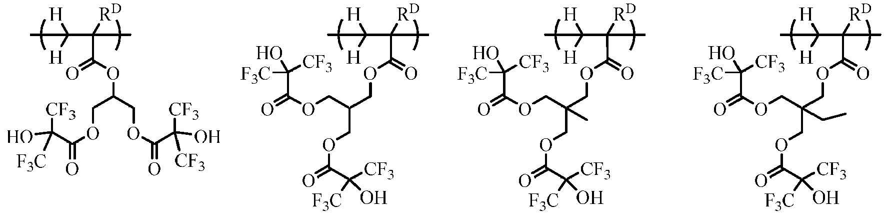

The repeat unit (D1) is preferably incorporated in an amount of 5 to 85 mol%, more preferably 15 to 80 mol% based on the overall repeat units of the fluorinated polymer. The repeat units (D2) to (D5), which may be used alone or in admixture, are preferably incorporated in an amount of 15 to 95 mol%, more preferably 20 to 85 mol% based on the overall repeat units of the fluorinated polymer.

The fluorinated polymer may comprise additional repeat units as well as the repeat units (D1) to (D5). Suitable additional repeat units include those described in USP 9,091,918 (JP-A 2014-177407, paragraphs [0046]-[0078]). When the fluorinated polymer comprises additional repeat units, their content is preferably up to 50 mol% based on the overall repeat units.

The fluorinated polymer may be synthesized by combining suitable monomers optionally protected with a protective group, copolymerizing them in the standard way, and effecting deprotection reaction if necessary. The copolymerization reaction is preferably radical polymerization or anionic polymerization though not limited thereto. For the polymerization reaction, reference may be made to JP-A 2004-115630.

The fluorinated polymer should preferably have a weight average molecular weight (Mw) of 2,000 to 50,000, and more preferably 3,000 to 20,000. A fluorinated polymer with a Mw of less than 2,000 allows acid diffusion, degrading resolution and detracting from age stability. A polymer with too high Mw has a reduced solubility in solvent, leading to coating defects. The fluorinated polymer preferably has a dispersity (Mw/Mn) of 1.0 to 2.2, more preferably 1.0 to 1.7.

The fluorinated polymer is preferably used in an amount of 0.01 to 30 parts, more preferably 0.1 to 20 parts by weight per 80 parts by weight of the base polymer.

### Surfactant

In the resist composition, any of surfactants commonly used for improving coating characteristics to the substrate may be added as an optional component. Numerous surfactants are known in the art and commercially available, for example, PF-636 (by Omnova Solutions) and FC-4430 (by 3M) and those described in JP-A 2004-115630. A choice may be made with reference to such patent documents. An appropriate amount of the surfactant used is 0 to 5 parts by weight per 80 parts by weight of the base polymer.

### Process

A further embodiment of the invention is a resist pattern forming process comprising the steps of applying the chemically amplified negative resist composition defined above onto a substrate to form a resist film thereon, exposing the resist film patternwise to high-energy radiation, and developing the exposed resist film in an alkaline developer to form a resist pattern.

In general, the resist composition is first applied onto a substrate such as a substrate for IC fabrication (e.g., Si, SiO₂, SiN, SiON, TiN, WSi, BPSG, SOG, organic antireflective coating, etc.) or a substrate for mask circuit fabrication (e.g., Cr, CrO, CrON, MoSi₂, SiO₂, etc.) by a suitable coating technique such as spin coating. The coating is prebaked on a hotplate preferably at a temperature of 60 to 150°C for 1 to 20 minutes, more preferably at 80 to 140°C for 1 to 10 minutes to form a resist film of 0.03 to 2 µm thick.

Then the resist film is exposed patternwise to high-energy radiation such as UV, deep UV, excimer laser (e.g., KrF, ArF), EUV, x-ray, γ-ray, synchrotron radiation or EB. Preferably EUV or EB is used for exposure.

On use of UV, deep UV, excimer laser, EUV, x-ray, γ-ray, or synchrotron radiation as the high-energy radiation, exposure is made through a mask having the desired pattern in a dose of preferably 1 to 200 mJ/cm², more preferably 10 to 200 mJ/cm². On use of EB, the desired pattern may be drawn by directly irradiating EB in a dose of preferably 1 to 300 µC/cm², more preferably 10 to 200 µC/cm².

The resist film is then baked (PEB) on a hotplate preferably at 60 to 150°C for 1 to 20 minutes, more preferably at 80 to 140°C for 1 to 10 minutes.

Thereafter the resist film is developed with a developer in the form of an aqueous base solution, for example, 0.1 to 5 wt%, preferably 2 to 3 wt% aqueous solution of tetramethylammonium hydroxide (TMAH) for 0.1 to 3 minutes, preferably 0.5 to 2 minutes by conventional techniques such as dip, puddle and spray techniques. In this way, a desired resist pattern is formed on the substrate.

After the resist film is formed, pure water rinsing (or post-soaking) may be performed for extracting the acid generator or the like from the film surface or washing away foreign particles. After the exposure, rinsing (or post-soaking) may be performed for removing any residual water from the resist film.

### EXAMPLES

Examples of the invention are given below by way of illustration and not by way of limitation. The abbreviation "pbw" is parts by weight; THF stands for tetrahydrofuran, DMF for dimethylformamide, and PGMEA for propylene glycol monomethyl ether acetate. Analytic instruments are as shown below.

| | |
|---|---|
| IR: | NICOLET 6700 by Thermo Fisher Scientific Inc. |
| ¹H-NMR: | ECA-500 by JEOL Ltd. |
| ¹³C-NMR: | ECA-500 by JEOL Ltd. |

### [1] Synthesis of alcohol compounds

### Example 1-1

### Synthesis of Crosslinker CA-1

### (1) Synthesis of Intermediate In-1

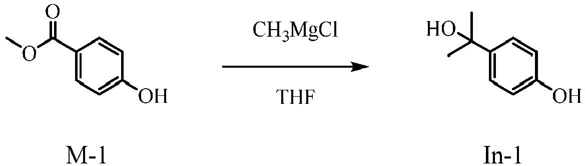

In a flask under nitrogen atmosphere, a solution of 210 g of M-1 in 500 g of THF was added dropwise to a Grignard reagent which had been prepared from 145.9 g (6 equivalents per equivalent of M-1) of magnesium, 3,000 g of THF and chloromethane, while maintaining the temperature in the flask (internal temperature, hereinafter) at 40 to 55°C. At the end of addition, the reaction system was aged for 3 hours at an internal temperature of 50°C. After aging, the reaction system was cooled, whereupon a mixture of 600 g of ammonium chloride and 1,800 g of 3.0 wt% hydrochloric acid aqueous solution was added dropwise to quench the reaction. This was followed by extraction with 2,000 mL of ethyl acetate, ordinary aqueous work-up, solvent distillation, and recrystallization from hexane, obtaining an Intermediate In-1 in white crystal form (amount 172.2 g, yield 82%).

### (2) Synthesis of Crosslinker CA-1

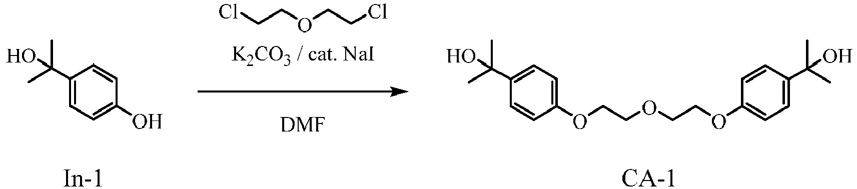

In nitrogen atmosphere, a flask was charged with 10.0 g of In-1, 13.6 g of potassium carbonate, 3.9 g of sodium iodide, and 50 g of DMF, which were stirred at an internal temperature of 60°C for 30 minutes. To the flask, 3.8 g of bis(2-chloroethyl) ether was added dropwise. At the end of addition, the reaction mixture was aged for 18 hours while maintaining the internal temperature of 60°C. After aging, 150 mL of ethyl acetate and 50 mL of 5 wt% sodium hydroxide aqueous solution were added to quench the reaction. This was followed by ordinary aqueous work-up and solvent distillation. The residue was dissolved in 10 g of dichloromethane. Subsequent decantation using hexane gave a compound CA-1 as oily matter (amount 5.5 g, yield 56%).

The compound was analyzed by spectroscopy. The data of infrared (IR) absorption are shown below. FIG. 1 shows the NMR spectrum (¹H-NMR in DMSO-d₆).
IR (D-ATR): v = 3405, 3040, 2972, 2928, 2874, 1610, 1583, 1512, 1456, 1413, 1363, 1297, 1245, 1179, 1134, 1064, 1012, 953, 864, 832, 731, 684, 560 cm⁻¹

### Examples 1-2 to 1-15

### Synthesis of Crosslinkers CA-2 to CA-15

Crosslinkers CA-2 to CA-15 were synthesized by similar organic synthesis methods.

### Comparative Examples 1-1 to 1-3

### Synthesis of Crosslinkers cCA-1 to cCA-3

The following compounds (commercially available from Tokyo Chemical Industry Co., Ltd.) were used as Crosslinkers cCA-1 to cCA-3.
cCA-1: 1,3,4,6-tetrakis(methoxymethyl)glycoluril
cCA-2: α,α'-dihydroxy-1,4-diisopropylbenzene
cCA-3: 4,4',4"-trihydroxytriphenylmethane

The structure of CA-2 to CA-15 and cCA-1 to cCA-3 is shown below.

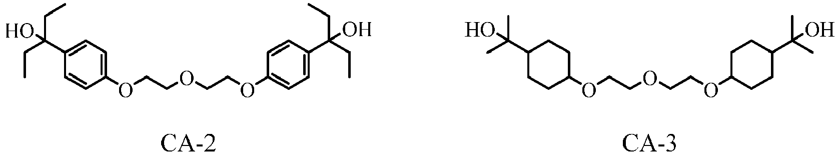

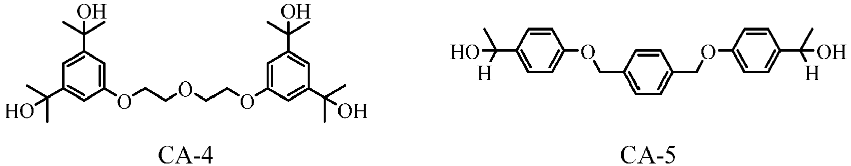

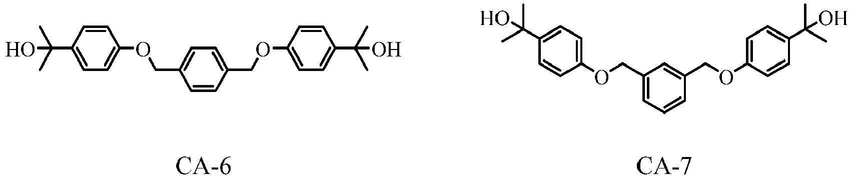

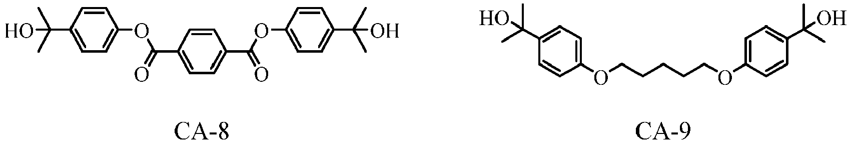

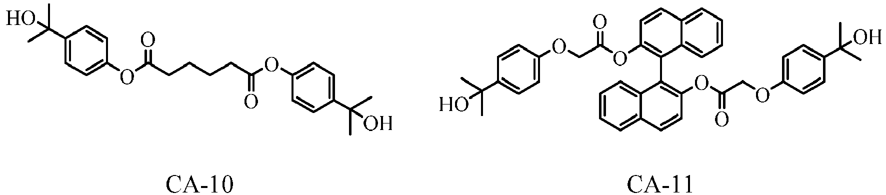

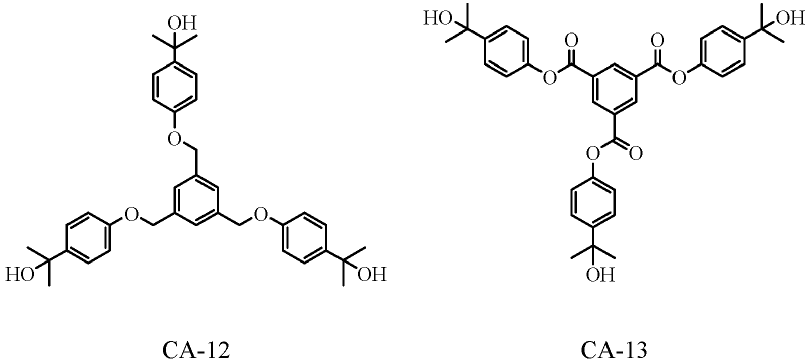

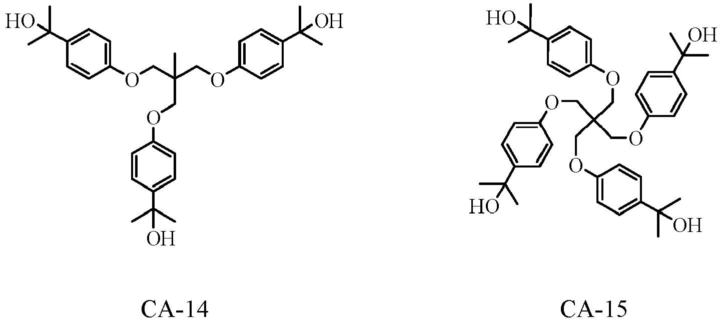

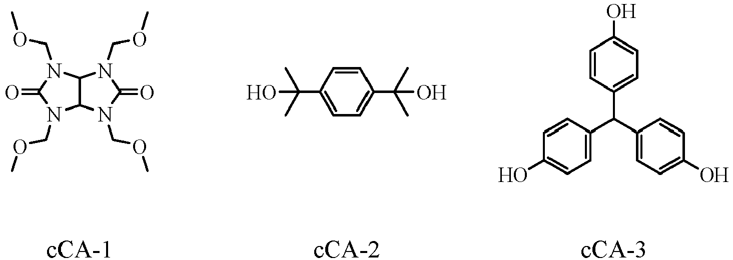

### [2] Synthesis of polymers

### Synthesis Example 1

### Synthesis of Polymer P-1

A 3-L flask was charged with 301.8 g of 4-acetoxystyrene, 190.7 g of acenaphthylene, 513 g of a 55.8 wt% PGMEA solution of 4-ethenyl-α,α-dimethylbenzenemethanol, and 814 g of toluene as solvent. The reactor was cooled at -70°C in a nitrogen atmosphere, after which vacuum pumping and nitrogen flow were repeated three times. The reactor was warmed up to room temperature, whereupon 40.5 g of 2,2'-azobis(2,4-dimethylvaleronitrile) (V-65 by Fuji Film Wako Chemicals) was added as polymerization initiator. The reactor was heated at 45°C whereupon reaction ran for 20 hours and then heated at 55°C whereupon reaction ran for a further 20 hours. The reaction solution was concentrated to one-half, which was added to 7,000 g of hexane for precipitation. The resulting precipitate was collected by filtration and dried at 40°C under reduced pressure, obtaining a white solid. The white solid was dissolved in a mixture of 300 g of methanol and 900 g of THF again, to which 170.5 g of 2-ethanolamine was added. The reaction mixture was heated at 60°C for 3 hours for deprotection reaction. The reaction solution was concentrated. The concentrate was dissolved in 1,500 g of ethyl acetate, whereupon the solution was subjected to one neutralization separatory washing with a mixture of 450 g of water and 85.3 g of acetic acid, one washing with a mixture of 450 g of water and 113.7 g of pyridine, and 4 times of separatory washing with 450 g of water. The upper layer, ethyl acetate solution was concentrated. The concentrate was dissolved in 850 g of acetone and added to 15 L of water for precipitation. The precipitate was filtered and dried in vacuum at 50°C for 40 hours, yielding 349.4 g of Polymer P-1 as white solid. On analysis by ¹H-NMR, ¹³C-NMR, and GPC using THF, Polymer P-1 was identified as below.

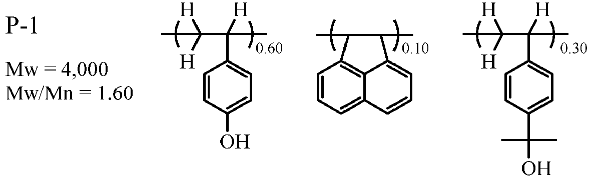

### Synthesis Example 2

### Synthesis of Polymer P-13

In nitrogen atmosphere, a 3000-mL dropping funnel was charged with 890 g of 50.0 wt% PGMEA solution of 4-hydroxystyrene, 47.7 g of acenaphthylene, 310 g of 54.7 wt% PGMEA solution of 4-(2-hydroxy-2-propyl)styrene, 87.0 g of triphenylsulfonium 1,1,3,3,3-pentafluoro-2-methacryloyloxypropane-1-sulfonate, 96.1 g of dimethyl 2,2'-azobis(2-methylpropionate) (V-601 by Fuji Film Wako Chemicals), and 360 g of γ-butyronitrile and 220 g of PGMEA as solvents to form a monomer solution. In nitrogen atmosphere, a 5000-mL flask was charged with 580 g of γ-butyronitrile, which was heated at 80°C. The monomer solution was added dropwise to the flask over 4 hours. At the end of addition, the solution was stirred for 18 hours for polymerization while maintaining the temperature of 80°C. The polymerization solution was cooled to room temperature and then added dropwise to 22.5 kg of diisopropyl ether, whereupon a white solid precipitated. After diisopropyl ether was decanted off, the white solid precipitate was dissolved in 2,250 g of acetone. The acetone solution was added dropwise to 22.5 kg of diisopropyl ether. The resulting white solid precipitate was collected by filtration. The white solid was dissolved in 2,250 g of acetone again, after which the acetone solution was added dropwise to 22.5 kg of water. The white solid precipitate was collected by filtration and dried at 40°C for 40 hours, yielding Polymer P-13 (amount 700 g). On analysis by ¹H-NMR, ¹³C-NMR, and GPC using DMF, Polymer P-13 was identified as below.

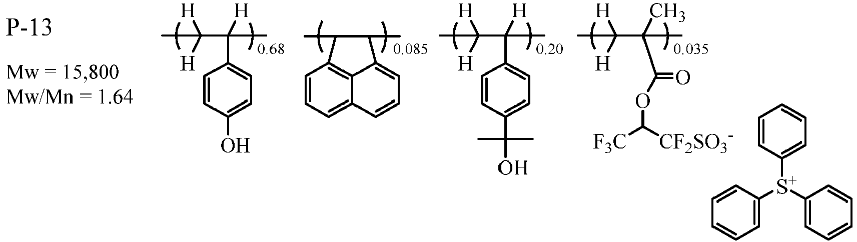

### Synthesis Examples 3 to 24

### Synthesis of Polymers P-2 to P-12 and P-14 to P-24

Polymers P-2 to P-12 and P-14 to P-24 were synthesized as in Synthesis Example 1 or 2 aside from changing the type and incorporation ratio (mol%) of monomers. The repeat units constituting Polymers P-1 to P-24 and their incorporation ratio are tabulated in Table 1. It is noted that the Mw of Polymers P-1 to P-12 and P-20 to P-23 is measured by GPC versus polystyrene standards using THF whereas the Mw of Polymers P-13 to P-19 and P-24 is measured by GPC versus polystyrene standards using DMF.

**Table 1**

| Polymer | Unit 1 | Incorporation ratio (mol%) | Unit 2 | Incorporation ratio (mol%) | Unit 3 | Incorporation ratio (mol%) | Unit 4 | Incorporation ratio (mol%) | Unit 5 | Incorporation ratio (mol%) | Mw | Mw/Mn |
|---|---|---|---|---|---|---|---|---|---|---|---|---|
| P-1 | A-1 | 60.0 | B-1 | 10.0 | C-1 | 30.0 | - | - | - | - | 4,000 | 1.60 |
| P-2 | A-1 | 60.0 | B-1 | 10.0 | C-1 | 30.0 | - | - | - | - | 2,800 | 1.53 |
| P-3 | A-1 | 70.0 | B-1 | 7.0 | C-2 | 23.0 | - | - | - | - | 3,600 | 1.63 |
| P-4 | A-1 | 70.0 | B-1 | 10.0 | C-3 | 20.0 | - | - | - | - | 3,900 | 1.65 |
| P-5 | A-1 | 55.0 | B-1 | 10.0 | C-4 | 35.0 | - | - | - | - | 4,400 | 1.64 |
| P-6 | A-1 | 45.0 | B-1 | 10.0 | C-5 | 45.0 | - | - | - | - | 4,600 | 1.58 |
| P-7 | A-1 | 50.0 | B-1 | 10.0 | C-5 | 40.0 | - | - | - | - | 4,900 | 1.63 |
| P-8 | A-1 | 50.0 | B-2 | 10.0 | C-1 | 40.0 | - | - | - | - | 4,100 | 1.67 |
| P-9 | A-1 | 50.0 | B-2 | 10.0 | C-1 | 40.0 | - | - | - | - | 3,000 | 1.64 |
| P-10 | A-1 | 55.0 | B-3 | 10.0 | C-1 | 35.0 | - | - | - | - | 4,000 | 1.63 |
| P-11 | A-2 | 70.0 | B-1 | 10.0 | C-1 | 20.0 | - | - | - | - | 4,100 | 1.65 |
| P-12 | A-2 | 75.0 | C-1 | 25.0 | - | - | - | - | - | - | 4,000 | 1.65 |
| P-13 | A-1 | 68.0 | B-1 | 8.5 | C-1 | 20.0 | E-1 | 3.5 | - | - | 15,800 | 1.64 |
| P-14 | A-1 | 61.0 | B-2 | 10.5 | C-1 | 25.0 | E-1 | 3.5 | - | - | 16,500 | 1.62 |
| P-15 | A-1 | 67.0 | B-1 | 10.0 | C-1 | 18.5 | E-2 | 4.5 | - | - | 14,000 | 1.63 |
| P-16 | A-1 | 67.0 | B-1 | 9.3 | C-1 | 20.0 | E-3 | 3.7 | - | - | 13,500 | 1.63 |
| P-17 | A-1 | 67.3 | B-1 | 10.0 | C-1 | 17.5 | E-4 | 5.2 | - | - | 13,200 | 1.64 |
| P-18 | A-1 | 64.1 | B-1 | 9.5 | C-1 | 22.0 | E-5 | 4.4 | - | - | 12,800 | 1.62 |
| P-19 | A-1 | 64.0 | B-1 | 10.0 | C-1 | 22.8 | E-6 | 3.2 | - | - | 13,500 | 1.63 |
| P-20 | A-2 | 70.0 | C-1 | 20.0 | D-1 | 10.0 | - | - | - | - | 5,500 | 1.65 |
| P-21 | A-2 | 68.0 | C-1 | 21.0 | D-2 | 11.0 | - | - | - | - | 5,800 | 1.69 |
| P-22 | A-3 | 69.0 | C-1 | 20.5 | D-3 | 10.5 | - | - | - | - | 7,000 | 1.68 |
| P-23 | A-3 | 70.0 | C-1 | 20.0 | D-4 | 10.0 | - | - | - | - | 6,400 | 1.70 |
| P-24 | A-1 | 57.5 | B-1 | 2.5 | C-1 | 30.0 | E-5 | 10.0 | - | - | 11,000 | 1.65 |

The structure of each repeat unit is shown below.

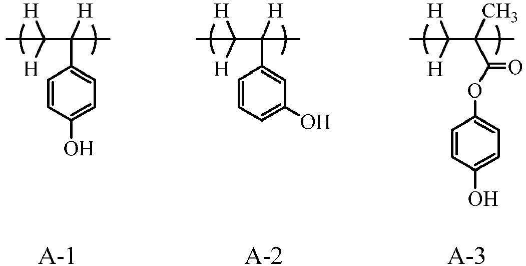

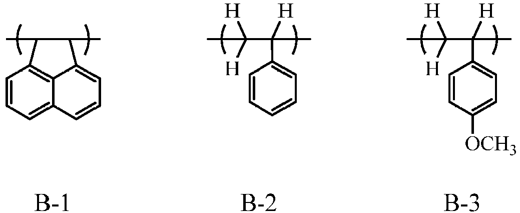

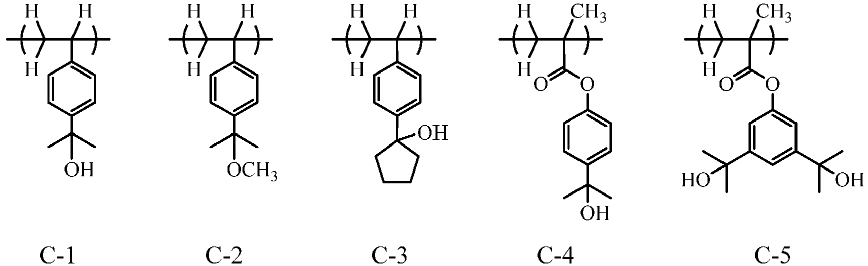

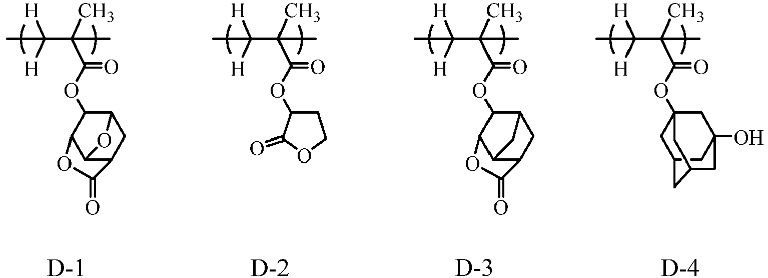

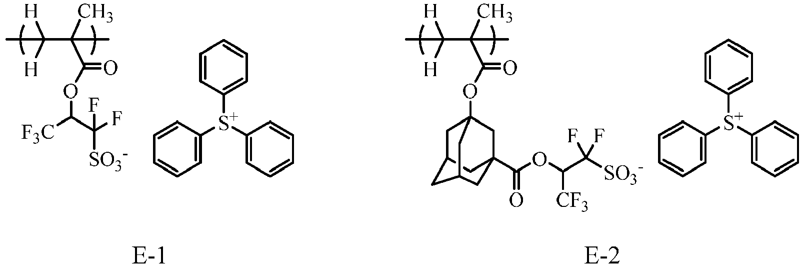

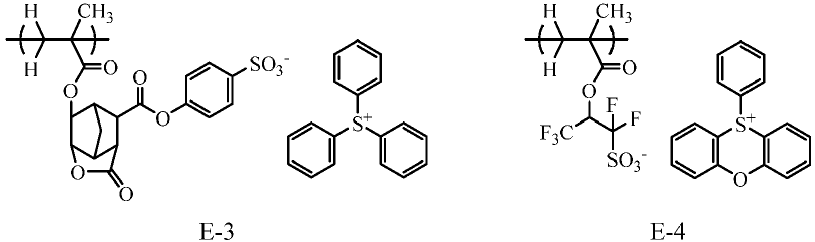

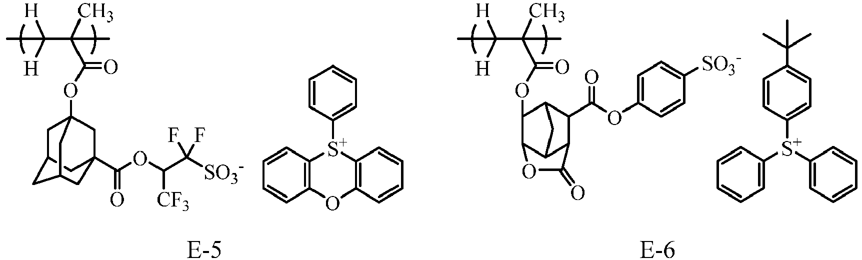

### [3] Preparation of chemically amplified negative resist compositions

### Examples 2-1 to 2-53 and Comparative Examples 2-1 to 2-13

Chemically amplified negative resist compositions R-1 to R-53 and comparative chemically amplified negative resist compositions cR-1 to cR-13 were prepared by dissolving selected components in an organic solvent according to the formulation shown in Tables 2 to 4, and filtering through a UPE filter and/or nylon filter with a pore size of 0.02 µm. The organic solvent used in R-1 to R-51 and cR-1 to cR-9 was a mixture of 1,204 pbw of PGMEA, 1,204 pbw of ethyl lactate (EL), and 1,606 pbw of propylene glycol monomethyl ether (PGME). The organic solvent used in R-52, R-53 and cR-10 to cR-13 was a mixture of 1,780 pbw of PGMEA, 1,920 pbw of EL, and 2,800 pbw of PGME.

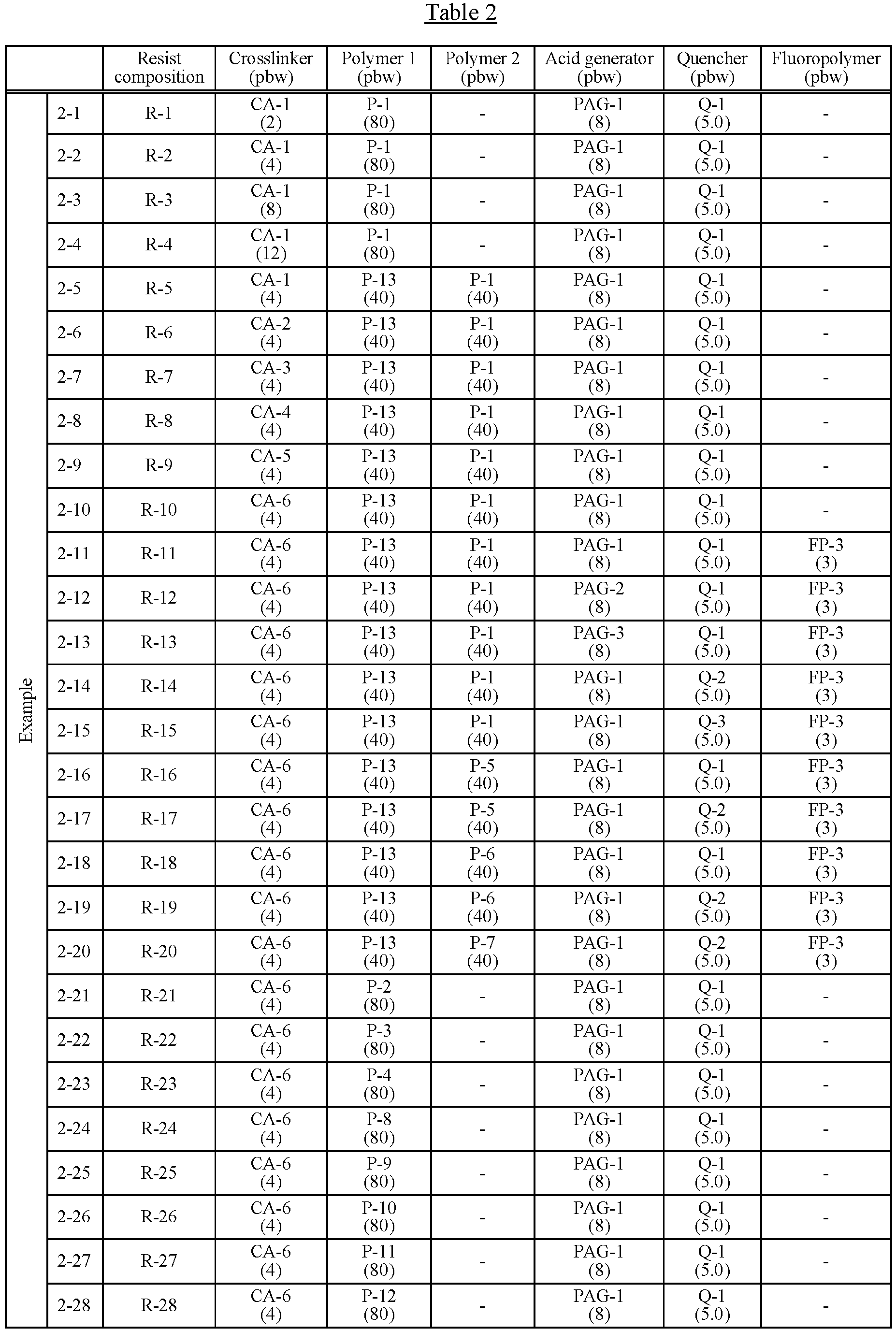

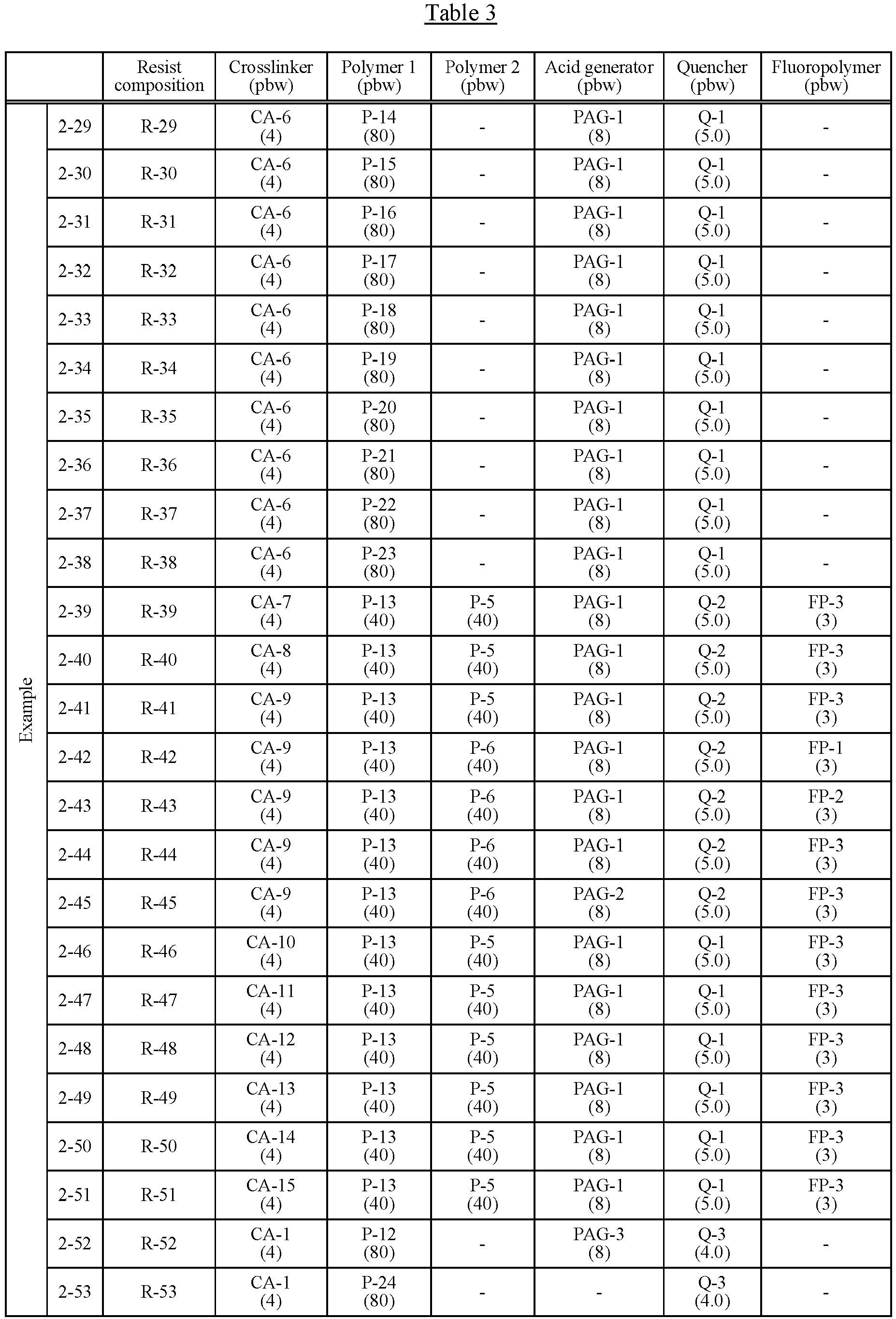

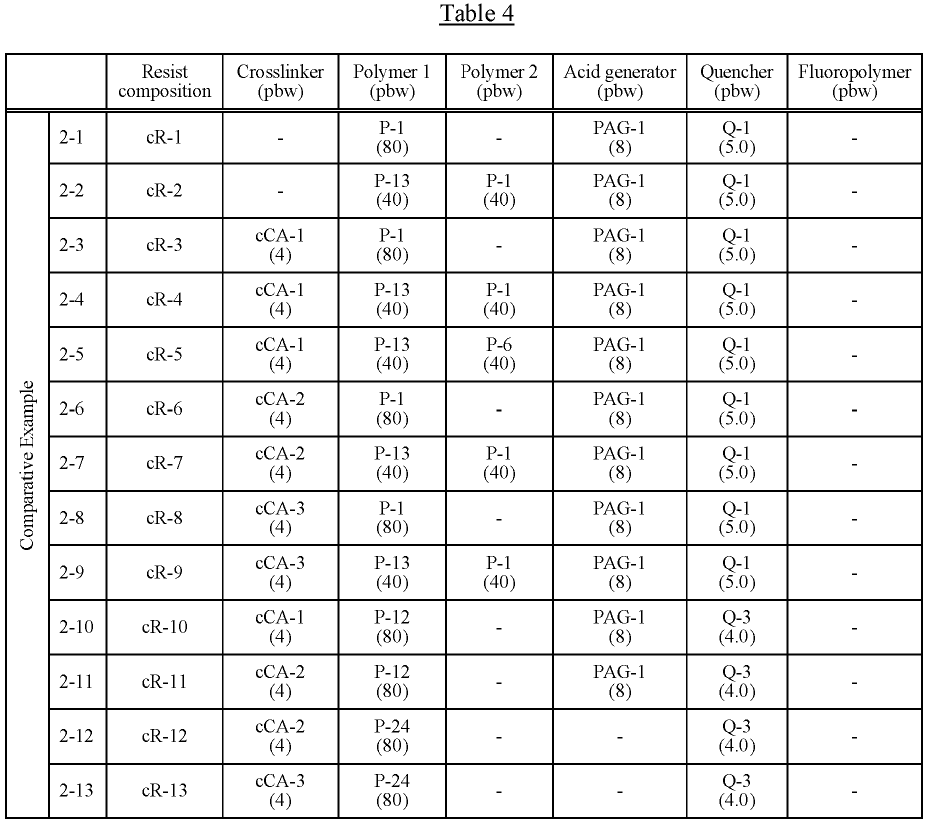

Notably, acid generators PAG-1 to PAG-3, fluorine-containing polymers FP-1 to FP-3, and quenchers Q-1 to Q-3 in Tables 2 to 4 are identified below.

### Acid generators PAG-1 to PAG-3:

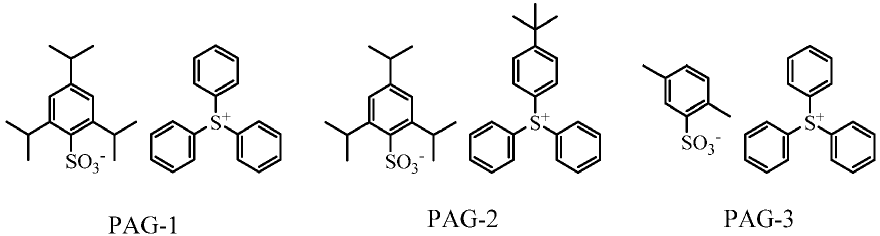

### Fluoropolymers FP-1 to FP-3:

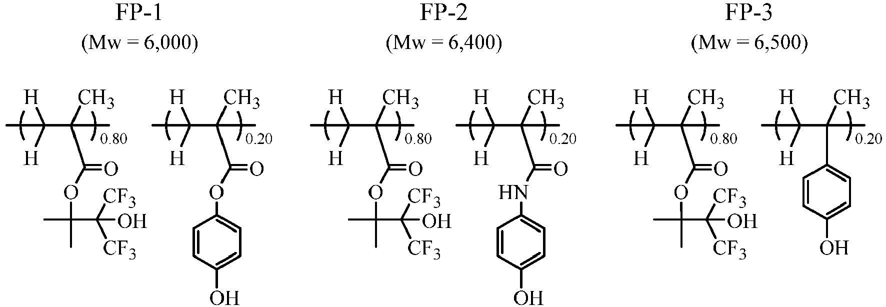

### Quenchers Q-1 to Q-3:

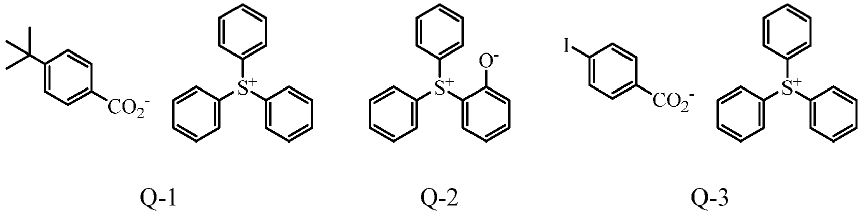

### [4] EB lithography test

### Examples 3-1 to 3-51 and Comparative Examples 3-1 to 3-9

Using a coater/developer system ACT-M (Tokyo Electron Ltd.), each of the negative resist compositions R-1 to R-51 and cR-1 to cR-9 was spin coated onto a mask blank of 152 mm squares having the outermost surface of chromium and prebaked on a hotplate at 110°C for 600 seconds to form a resist film of 80 nm thick. The thickness of the resist film was measured by an optical film thickness measurement system Nanospec (Nanometrics Inc.). Measurement was made at 81 points in the plane of the blank substrate excluding a peripheral band extending 10 mm inward from the blank periphery, and an average film thickness and a film thickness range were computed therefrom.

The resist film was exposed to EB using an EB writer system EBM-5000Plus (NuFlare Technology Inc., accelerating voltage 50 kV), then baked (PEB) at 120°C for 600 seconds, and developed in a 2.38 wt% TMAH aqueous solution, thereby yielding a negative line-and-space (LS) pattern. The resist pattern was evaluated as follows, with the results shown in Tables 5 to 7.

### Evaluation of optimum dose

The patterned mask blank was observed under a top-down scanning electron microscope (TD-SEM). The optimum exposure was defined as an exposure dose (µC/cm²) which provided a 1:1 resolution of a 200-nm 1:1 LS pattern.

### Evaluation of LER

The 200-nm LS pattern printed by exposure at the optimum dose was observed under SEM. For each of the edges of 32 lines of the LS pattern, edge detection was carried out at 80 points, from which a 3-fold value (3σ) of standard deviation (σ) was determined and reported as LER (nm).

### Evaluation of CDU

For the 200-nm LS pattern printed by exposure at the optimum dose, the size was measured at 144 spots within the plane of the blank substrate, from which a 3-fold value (3σ) of standard deviation (σ) was determined and reported as CDU. A smaller value of 3σ indicates a LS pattern having improved CDU.

### Evaluation of maximum resolution

The minimum size of IL printed at an exposure dose that allows an actual size of 200 nm to be resolved from an isolated line (IL) having a design size of 200 nm is regarded as IL resolution (maximum resolution). The minimum size of IS printed at an exposure dose that allows an actual size of 200 nm to be resolved from an isolated space (IS) having a design size of 200 nm is regarded as IS resolution (maximum resolution). It is noted that the IL resolution is the resolution of a single isolated line pattern and the IS resolution is the resolution of a single isolated space pattern.

**Table 5**

| | | Resist composition | Optimum dose (µC/cm²) | LER (nm) | CDU (nm) | IL/IS maximum resolution (nm) |
|---|---|---|---|---|---|---|
| | 3-1 | R-1 | 59 | 3.8 | 1.9 | 40/40 |
| | 3-2 | R-2 | 52 | 3.7 | 1.8 | 35/37 |
| | 3-3 | R-3 | 44 | 3.9 | 1.9 | 35/37 |
| | 3-4 | R-4 | 38 | 4.3 | 2.0 | 40/40 |
| | 3-5 | R-5 | 48 | 3.7 | 2.0 | 35/37 |
| | 3-6 | R-6 | 48 | 3.6 | 2.0 | 35/37 |
| | 3-7 | R-7 | 49 | 3.6 | 1.8 | 35/37 |
| | 3-8 | R-8 | 48 | 3.7 | 1.8 | 35/37 |
| | 3-9 | R-9 | 48 | 3.7 | 1.8 | 35/37 |
| | 3-10 | R-10 | 50 | 3.7 | 1.8 | 35/37 |
| | 3-11 | R-11 | 49 | 3.9 | 1.8 | 35/37 |
| | 3-12 | R-12 | 49 | 3.9 | 1.9 | 37/37 |
| | 3-13 | R-13 | 48 | 3.9 | 1.8 | 37/37 |
| | 3-14 | R-14 | 50 | 3.5 | 1.7 | 37/35 |
| | 3-15 | R-15 | 49 | 3.7 | 1.8 | 37/37 |
| | 3-16 | R-16 | 48 | 3.4 | 1.6 | 35/35 |
| | 3-17 | R-17 | 49 | 3.5 | 1.7 | 35/35 |
| Example | 3-18 | R-18 | 48 | 3.3 | 1.8 | 35/37 |
| | 3-19 | R-19 | 49 | 3.1 | 1.8 | 35/37 |
| | 3-20 | R-20 | 50 | 3.2 | 1.8 | 35/32 |
| | 3-21 | R-21 | 49 | 3.7 | 1.9 | 35/37 |
| | 3-22 | R-22 | 49 | 3.7 | 1.8 | 35/37 |
| | 3-23 | R-23 | 50 | 3.6 | 1.7 | 35/37 |
| | 3-24 | R-24 | 51 | 3.7 | 1.8 | 35/37 |
| | 3-25 | R-25 | 49 | 3.6 | 1.9 | 35/37 |
| | 3-26 | R-26 | 49 | 3.5 | 1.8 | 35/37 |
| | 3-27 | R-27 | 48 | 3.6 | 1.8 | 35/37 |
| | 3-28 | R-28 | 50 | 3.7 | 1.8 | 35/37 |
| | 3-29 | R-29 | 47 | 3.5 | 2.0 | 37/35 |
| | 3-30 | R-30 | 47 | 3.6 | 1.9 | 37/35 |
| | 3-31 | R-31 | 48 | 3.5 | 2.0 | 37/35 |
| | 3-32 | R-32 | 47 | 3.6 | 1.9 | 37/35 |
| | 3-33 | R-33 | 47 | 3.5 | 2.0 | 37/35 |
| | 3-34 | R-34 | 49 | 3.7 | 2.0 | 37/35 |
| | 3-35 | R-35 | 47 | 3.7 | 2.0 | 35/37 |

**Table 6**

| | | Resist composition | Optimum dose (µC/cm²) | LER (nm) | CDU (nm) | IL/IS maximum resolution (nm) |
|---|---|---|---|---|---|---|
| | 3-36 | R-36 | 49 | 3.5 | 1.9 | 35/37 |
| | 3-37 | R-37 | 48 | 3.6 | 2.0 | 37/35 |
| | 3-38 | R-38 | 51 | 3.5 | 1.9 | 37/35 |
| | 3-39 | R-39 | 50 | 3.4 | 1.8 | 35/37 |
| | 3-40 | R-40 | 50 | 3.4 | 1.8 | 35/37 |
| | 3-41 | R-41 | 49 | 3.5 | 1.7 | 35/37 |
| | 3-42 | R-42 | 50 | 3.4 | 1.6 | 35/35 |
| Example | 3-43 | R-43 | 50 | 3.5 | 1.8 | 35/35 |
| | 3-44 | R-44 | 49 | 3.4 | 1.7 | 35/35 |
| | 3-45 | R-45 | 50 | 3.5 | 1.8 | 37/35 |
| | 3-46 | R-46 | 51 | 3.6 | 1.8 | 35/35 |
| | 3-47 | R-47 | 52 | 3.7 | 1.9 | 35/35 |
| | 3-48 | R-48 | 50 | 3.5 | 1.9 | 37/35 |
| | 3-49 | R-49 | 51 | 3.7 | 1.8 | 37/35 |
| | 3-50 | R-50 | 50 | 3.6 | 1.8 | 37/35 |
| | 3-51 | R-51 | 51 | 3.6 | 1.9 | 37/35 |

**Table 7**

| | | Resist composition | Optimum dose (µC/cm²) | LER (nm) | CDU (nm) | IL/IS maximum resolution (nm) |
|---|---|---|---|---|---|---|
| | 3-1 | cR-1 | 75 | 4.6 | 2.5 | 45/50 |
| | 3-2 | cR-2 | 72 | 4.5 | 2.4 | 45/45 |
| | 3-3 | cR-3 | 65 | 4.7 | 2.5 | 45/45 |
| | 3-4 | cR-4 | 62 | 4.6 | 2.3 | 45/45 |
| Comparative Example | 3-5 | cR-5 | 63 | 4.4 | 2.2 | 40/45 |
| | 3-6 | cR-6 | 67 | 4.5 | 2.6 | 45/50 |
| | 3-7 | cR-7 | 65 | 4.5 | 2.5 | 45/45 |
| | 3-8 | cR-8 | 66 | 4.6 | 2.4 | 45/50 |
| | 3-9 | cR-9 | 65 | 4.5 | 2.5 | 45/45 |

### [5] EUV lithography test

### Examples 4-1, 4-2 and Comparative Examples 4-1 to 4-4

The negative resist compositions R-52, R-53, cR-10 to cR-13 were baked at 200°C for water removal. Each resist composition was spin coated on a silicon substrate (which had underwent vapor prime treatment with hexamethyldisilazane at 100°C for 90 seconds) and prebaked on a hotplate at 110°C for 60 seconds to form a resist film of 30 nm thick.

Using an EUV scanner NXE3300 (ASML, NA 0.33, dipole 90), the resist film was exposed to EUV. The resist film was baked (PEB) on a hotplate at 120°C for 60 seconds and puddle developed in a 2.38 wt% TMAH aqueous solution for 60 seconds, obtaining a negative LS pattern. The resist pattern was evaluated as follows, with the results shown in Table 8.

### Evaluation of optimum dose

The pattern-bearing wafer was observed under TD-SEM. The optimum dose was defined as an exposure dose (mJ/cm²) which provided a 1:1 resolution of a 22-nm 1:1 LS pattern, and reported as sensitivity.

### Evaluation of resolution

The minimum size printed at the optimum dose is reported as resolution.

### Evaluation of LWR

A 22-nm LS pattern printed at the optimum dose was observed under CD-SEM (CG-5000 by Hitachi High Technologies Corp.). The line width was measured at 30 points, from which a 3-fold value (3σ) of standard deviation (σ) was determined and reported as LWR (nm).

### Evaluation of pattern profile

The profile of patterns printed at the optimum dose Eop was compared. A resist film providing a pattern of rectangular profile and perpendicular sidewall is evaluated "good." A resist film providing a pattern of tapered profile with remarkably inclined sidewall or top-rounded profile due to top loss is evaluated "NG."

**Table 8**

| | | Resist composition | Sensitivity (mJ/cm²) | Resolution (nm) | LWR (nm) | Profile |
|---|---|---|---|---|---|---|
| Example | 4-1 | R-52 | 38 | 18 | 4.2 | good |
| | 4-2 | R-53 | 40 | 18 | 4.5 | good |
| Comparative Example | 4-1 | cR-10 | 48 | 23 | 5.8 | NG |
| | 4-2 | cR-11 | 50 | 21 | 5.7 | NG |
| | 4-3 | cR-12 | 44 | 26 | 5.5 | NG |
| | 4-4 | cR-13 | 46 | 22 | 5.4 | NG |

As seen from Tables 5 to 8, the chemically amplified negative resist compositions within the scope of the invention show excellent lithography performance factors including sensitivity, CDU, resolution, and roughness when processed by the EB or EUV lithography.

Japanese Patent Application No. 2020-213114 is incorporated herein by reference.

Although some preferred embodiments have been described, many modifications and variations may be made thereto in light of the above teachings. It is therefore to be understood that the invention may be practiced otherwise than as specifically described without departing from the scope of the appended claims.

## Claims

1. A crosslinker comprising an alcohol compound of any of the following formulae

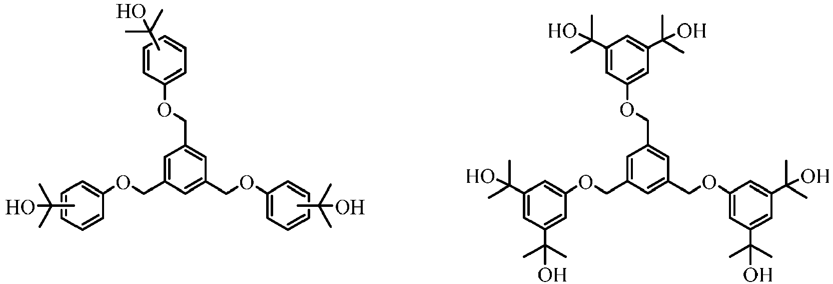

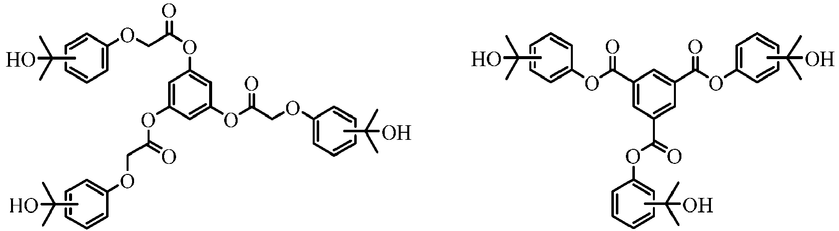

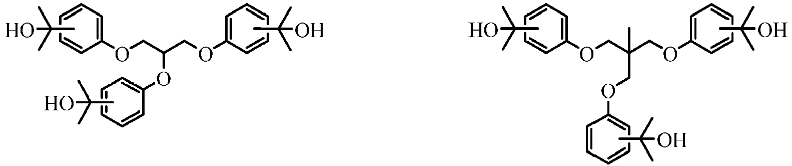

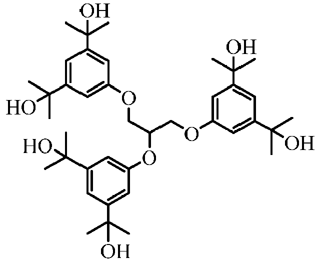

2. A chemically amplified negative resist composition comprising the crosslinker of claim 1.

3. The resist composition of claim 2, further comprising a base polymer comprising repeat units having the formula (B1) and repeat units having the formula (B2): 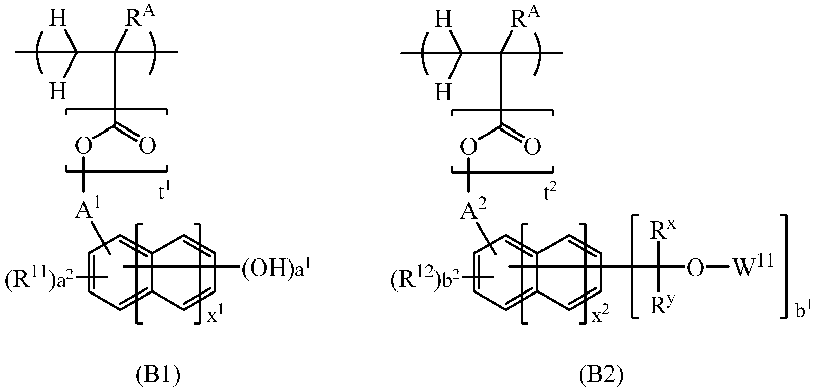 wherein R^{A} is hydrogen, fluorine, methyl or trifluoromethyl,
R¹¹ and R¹² are each independently halogen, an optionally halogenated C₁-C₆ saturated hydrocarbyl group, optionally halogenated C₂-C₈ saturated hydrocarbylcarbonyloxy group, or optionally halogenated C₁-C₆ saturated hydrocarbyloxy group,
A¹ and A² are each independently a single bond or a C₁-C₁₀ saturated hydrocarbylene group in which a constituent -CH₂- may be replaced by -O-,
W¹¹ is hydrogen, a C₁-C₁₀ aliphatic hydrocarbyl group or an optionally substituted aryl group, a constituent -CH₂- in the aliphatic hydrocarbyl group may be replaced by -O-, -C(=O)-, -O-C(=O)- or -C(=O)-O-,
R^{x} and R^{y} are each independently hydrogen or a C₁-C₁₅ saturated hydrocarbyl group which may be substituted with a hydroxy moiety or saturated hydrocarbyloxy moiety, or an optionally substituted aryl group, R^{x} and R^{y} are not hydrogen at the same time, and R^{x} and R^{y} may bond together to form a ring with the carbon atom to which they are attached,
t¹ and t² are each independently 0 or 1, x¹ and x² are each independently an integer of 0 to 2, a¹ and b¹ are each independently an integer of 1 to 3, a² is an integer satisfying 0 ≤ a² ≤ 5+2x¹-a¹, and b² is an integer satisfying 0 ≤ b² ≤ 5+2x²-b¹.

4. The resist composition of claim 3 wherein the base polymer further comprises repeat units of at least one type selected from repeat units having the formula (B3) and repeat units having the formula (B4): 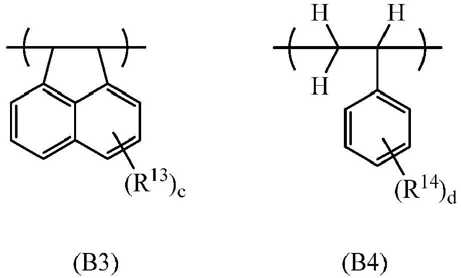 wherein R¹³ is a hydroxy group, halogen atom, acetoxy group, an optionally halogenated C₁-C₈ alkyl group, an optionally halogenated C₁-C₈ saturated hydrocarbyloxy group, an optionally halogenated C₂-C₈ saturated hydrocarbylcarbonyl group, or an optionally halogenated C₂-C₈ saturated hydrocarbylcarbonyloxy group, with the proviso that R¹³ is exclusive of an acid labile group,
R¹⁴ is a halogen atom, acetoxy group, an optionally halogenated C₁-C₈ alkyl group, an optionally halogenated C₁-C₈ saturated hydrocarbyloxy group, an optionally halogenated C₂-C₈ saturated hydrocarbylcarbonyl group, or an optionally halogenated C₂-C₈ saturated hydrocarbylcarbonyloxy group, with the proviso that R¹⁴ is exclusive of an acid labile group,
c and d are each independently an integer of 0 to 4.

5. The resist composition of claim 3 or 4 wherein the base polymer further comprises repeat units of at least one type selected from repeat units having the formulae (B5), (B6) and (B7): 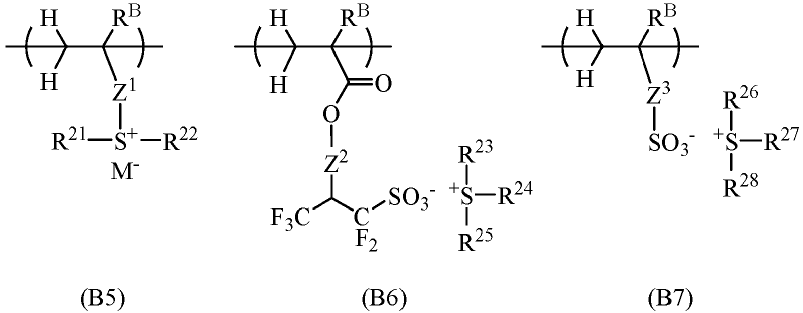 wherein R^{B} is each independently hydrogen or methyl,
Z¹ is a single bond, a C₁-C₆ aliphatic hydrocarbylene group, phenylene group, naphthylene group, or C₇-C₁₈ group obtained by combining the foregoing, or -O-Z¹¹-, -C(=O)-O-Z¹¹- or -C(=O)-NH-Z¹¹-, Z¹¹ is a C₁-C₆ aliphatic hydrocarbylene group, phenylene group, naphthylene group, or C₇-C₁₈ group obtained by combining the foregoing, which may contain a carbonyl moiety, ester bond, ether bond or hydroxy moiety,
Z² is a single bond or -Z²¹-C(=O)-O-, Z²¹ is a C₁-C₂₀ hydrocarbylene group which may contain a heteroatom,
Z³ is a single bond, methylene, ethylene, phenylene, fluorinated phenylene, trifluoromethyl-substituted phenylene group, -O-Z³¹-, -C(=O)-O-Z³¹-, or -C(=O)-NH-Z³¹-, Z³¹ is a C₁-C₁₀ aliphatic hydrocarbylene group, phenylene group, fluorinated phenylene group, trifluoromethyl-substituted phenylene group, or C₇-C₂₀ group obtained by combining the foregoing, which may contain a carbonyl moiety, ester bond, ether bond or hydroxy moiety,
R²¹ to R²⁸ are each independently halogen or a C₁-C₂₀ hydrocarbyl group which may contain a heteroatom, a pair of R²¹ and R²², R²³ and R²⁴ or R²⁶ and R²⁷ may bond together to form a ring with the sulfur atom to which they are attached, and
M⁻ is a non-nucleophilic counter ion.

6. The resist composition of claim 5 wherein the base polymer comprises a polymer containing repeat units having formula (B 1) and repeat units having formula (B2), but not repeat units having formula (B5), repeat units having formula (B6), and repeat units having formula (B7).

7. The resist composition of any one of claims 2 to 6, further comprising an organic solvent.

8. The resist composition of any one of claims 2 to 7, further comprising an acid generator.

9. The resist composition of any one of claims 2 to 8, further comprising a quencher.

10. The resist composition of any one of claims 2 to 9, further comprising a fluorinated polymer containing a polymer comprising repeat units having formula (D 1) and repeat units of at least one type selected from repeat units having formulae (D2) to (D5): 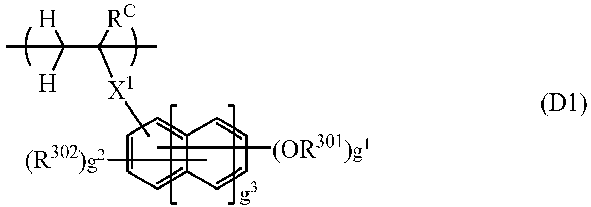 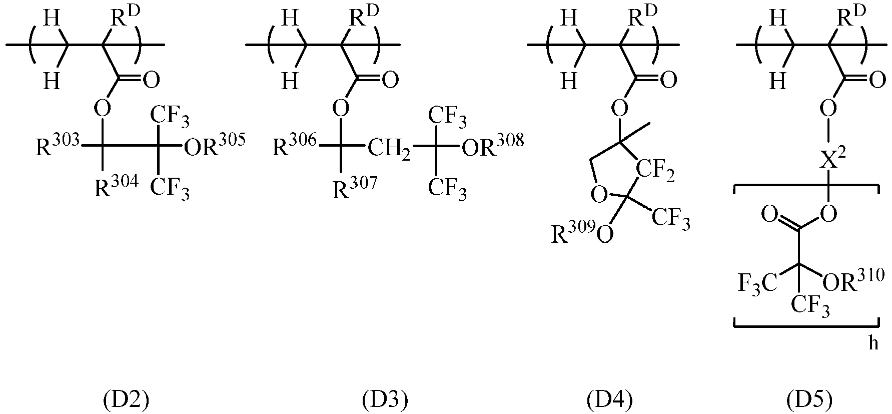 wherein R^{C} is each independently hydrogen or methyl,
R^{D} is each independently hydrogen, fluorine, methyl or trifluoromethyl,
R³⁰¹ is hydrogen or a C₁-C₅ straight or branched hydrocarbyl group in which a heteroatom-containing moiety may intervene in a carbon-carbon bond,
R³⁰² is a C₁-C₅ straight or branched hydrocarbyl group in which a heteroatom-containing moiety may intervene in a carbon-carbon bond,
R³⁰³, R³⁰⁴, R³⁰⁶ and R³⁰⁷ are each independently hydrogen or a C₁-C₁₀ saturated hydrocarbyl group,
R³⁰⁵, R³⁰⁸, R³⁰⁹ and R³¹⁰ are each independently hydrogen, a C₁-C₁₅ hydrocarbyl group, C₁-C₁₅ fluorinated hydrocarbyl group, or acid labile group, when R³⁰⁵, R³⁰⁸, R³⁰⁹ or R³¹⁰ is a hydrocarbyl or fluorinated hydrocarbyl group, an ether bond or carbonyl moiety may intervene in a carbon-carbon bond,
g¹ is an integer of 1 to 3, g² is an integer meeting 0 ≤ g² ≤ 5+2g³-g¹, g³ is 0 or 1, h is an integer of 1 to 3,
X' is a single bond, -C(=O)-O- or -C(=O)-NH-, and
X² is a C₁-C₂₀ (h+1)-valent hydrocarbon group or C₁-C₂₀ (h+1)-valent fluorinated hydrocarbon group.

11. A process for forming a resist pattern comprising the steps of applying the chemically amplified negative resist composition of any one of claims 2 to 10 onto a substrate to form a resist film thereon, exposing patternwise the resist film to high-energy radiation, and developing the exposed resist film in an alkaline developer.

12. The process of claim 11 wherein the high-energy radiation is EB or EUV of wavelength 3 to 15 nm.
